(19) Europäisches Patentamt / European Patent Office / Office européen des brevets

(11) **EP 4 122 489 A1**

(12) **EUROPEAN PATENT APPLICATION**
published in accordance with Art. 153(4) EPC

(43) Date of publication:
**25.01.2023 Bulletin 2023/04**

(21) Application number: **21776118.8**

(22) Date of filing: **24.03.2021**

(51) International Patent Classification (IPC):
*A61K 38/43* (2006.01)     *A61K 38/49* (2006.01)
*A61P 25/28* (2006.01)

(52) Cooperative Patent Classification (CPC):
**A61K 38/43; A61K 38/49; A61P 25/28**

(86) International application number:
**PCT/CN2021/082720**

(87) International publication number:
**WO 2021/190562 (30.09.2021 Gazette 2021/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.03.2020 CN 202010212897**

(71) Applicant: **Talengen International Limited
Hong Kong 999077 (HK)**

(72) Inventor: **LI, Jinan
Beijing 100089 (CN)**

(74) Representative: **karo IP
karo IP Patentanwälte
Kahlhöfer Rößler Kreuels PartG mbB
Postfach 32 01 02
40416 Düsseldorf (DE)**

Remarks:
The complete document including Reference Table(s) and the Sequence Listing(s) can be downloaded from the EPO website

(54) **METHOD AND DRUG FOR PROMOTING DEGRADATION OF MISFOLDED PROTEIN AND AGGREGATE THEREOF**

(57)     Provided in the present invention is a method for promoting the degradation of a misfolded protein and an aggregate thereof. The method includes administrating a therapeutically effective amount of plasminogen activation pathway components to a subject. Further provided in the present invention are a drug containing the plasminogen activation pathway components, a pharmaceutical composition, a product and a kit, which are used to prevent and treat diseases caused by the misfolded protein and/or the aggregate thereof.

EP 4 122 489 A1

**Description**

FIELD OF THE INVENTION

The present invention relates to a method for promoting the degradation of a misfolded protein and an aggregate thereof, including administering an effective amount of plasminogen activation pathway components or a related compound thereof, for example, plasminogen, to a subject to promote the degradation of the misfolded protein and the aggregate thereof, and to prevent and treat diseases caused by the misfolded protein and/or the aggregate thereof.

BACKGROUND OF THE INVENTION

Diseases caused by protein misfolding or aggregates thereof include the Alzheimer's disease, the Parkinson's disease, amyotrophic lateral sclerosis, polyglutamine diseases and prion diseases [Creutzfeldt-Jakob Disease (CJD), Gerstmann syndrome (GSS), fatal sporadic or familial isomnia and Kuru] and the like. Although causes of the diseases are different, and each disease has its own selective lesion sites and different gene mapping, the diseases have common pathological characteristics, namely, degeneration and death of neurons in specific regions and intraneuronal inclusions formed by aggregation of mutant proteins, such as senile plaques and neurofibrillary tangles in the brains of patients with the Alzheimer's disease; residual eosinophilic Lewy bodies in dopaminergic neurons of patients with the Parkinson's disease, Bunina bodies in spinal cord and brainstem neurons of patients with amyotrophic lateral sclerosis; affected intraneuronal inclusions of patients with the polyglutamine diseases and the like. Recent studies suggest that degeneration, death and inclusion body formation of these neurons are closely related to the aggregates formed by a misfolded protein.

Recent studies have demonstrated that intracellular a misfolded protein may be refolded by molecular chaperones such as heat shock proteins and degraded by a ubiquitin-proteasome system (Rubinsztein DC. The roles of intracellular protein-degradation pathways in neurodegen-eration, Nature, 2006, 443(7113): 780-786). After a misfolded protein is overproduced beyond their degradability or the proteasome function itself is impaired, a misfolded protein and the aggregates thereof are selectively mediated and is ubiquitinated mainly by polyubiquitin chains linked by K-63; and the ubiquitinated proteins and aggregates thereof interact with histone deacetylase 6 (HDAC6) (a microtubule-related α-tubulin deacetylase); and the HDAC6 mediates inclusion body-protein aggregates reversely transported to the periphery of the center of microtubule tissues along microtubules by dynein motors. This process is known as a protein aggregate pathway. Studies have shown that formation of the protein aggregates may be a protective mechanism for the corresponding diseases and may have the effect of recruiting potentially cytotoxic a misfolded protein and an aggregate thereof to form protein aggregates, so as to weaken their cytotoxic effects.

Alzheimer's disease and protein aggregates: the pathological characteristics of the Alzheimer's disease are extensive formation of extracellular Aβ plaques and intracellular neurofibrillary tangles (Braak H, Braak E. Evolution of neuronal changes in the course of Alzheimer's disease, Neural Transm Suppl, 1998, 53: 127-140). Results of a large number of studies have shown that the Alzheimer's disease is associated with the aggregate pathway.

The Parkinson's disease is a common neurodegenerative disease mainly characterized by selective loss of dopaminergic neurons and appearance of Lewy bodies (Spillantini MG, Schmidt ML, Lee VM, et al. Alphasynuclein in Lewy bodies, Nature, 1997, 3 88(6645): 839-840). The disease is associated with formation of inclusion bodies. The major protein component of Lewy bodies in the sporadic Parkinson's disease is α-synuclein.

The polyglutamine diseases are delayed and progressive neurodegenerative diseases caused by the abnormal repetition of a CAG sequence encoding glutamine, such as the Huntington's disease, spinocerebellar ataxia type 3, dentatorubral-pallidoluysian atrophy and the like. In the Huntington's disease, the disease is caused if there are more than 37 uninterrupted glutamines in a polyglutamine branch chain in a huntingtin exon 1.

The main pathological characteristics of the patients with the amyotrophic lateral sclerosis are formation of Bunina bodies, skein-like inclusions and Lewy body-like inclusions (Mizuno Y, Fujita Y, Takatama M, et al. Peripherin partially localizes in Bunina bodies in amyotrophic lateral sclerosis, J Neurol Sci, 2011, 302(1/2): 14-18), and the specific formation mechanism of the amyotrophic lateral sclerosis remains unclear.

In addition, the following diseases have been reported in the literature to be associated with misfolded/misconfigured/abnormally aggregated proteins as follows: cystic fibrosis versus a cystic fibrosis transmembrane conductance regulator (CFTR), emphysema and liver injury versus α1-antitryspin, the Parkinson's disease versus Parkin proteins, cataracts versus crystallin, familial amyloidosis versus transthyretin, short-chain acyl-CoA dehydrogenase (SCAD) deficiency versus a short-chain acyl-CoA dehydrogenase SCAD variant, familial hypercholesterolemia versus a low density lipoprotein receptor, the Huntington's disease versus huntingtin, the amyotrophic lateral sclerosis versus neurofilament protein, the amyotrophic lateral sclerosis versus peripherin, motor neuron diseases versus α-internexin, diabetes versus an islet amyloid polypeptide, dialysis-related amyloidosis versus β2-microglobulin, amyloidosis versus a serum amyloid A protein, amyloidosis versus immunoglobulin light chains, amyloidosis-related human diseases versus human lysozyme, amyloidosis versus α-lactalbumin, amyloidosis versus apolipoprotein E, amyloidosis versus apolipoprotein J.

At present, promoting refolding of a misfolded protein or the degradation of proteasome is still a good method for treating the neurodegenerative diseases, and it is necessary to find relevant drugs to provide an effective way for clinical treatment of the neurodegenerative diseases.

SUMMARY OF THE INVENTION

The present invention finds that plasminogen can promote the recovery of memory function in patients with the Alzheimer's disease, improve cognitive ability, significantly reduce and relieve various clinical symptoms and physical signs of patients with the Alzheimer's disease, and prevent and treat the Alzheimer's disease.

Specifically, the present invention relates to the following items.

1. In an aspect, the present invention relates to a method for promoting the degradation of a misfolded protein and an aggregate thereof, including: administering a therapeutically effective amount of one or more compounds to a subject. The one or more compounds are selected from: plasminogen activation pathway components, a compound capable of directly activating plasminogen or indirectly activating plasminogen by activating plasminogen activation pathway upstream components, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor. In an aspect, the present invention relates to use of one or more compounds in preparation of a drug for promoting the degradation of a misfolded protein and an aggregate thereof. The one or more compounds are selected from: plasminogen activation pathway components, a compound capable of directly activating plasminogen or indirectly activating plasminogen by activating plasminogen activation pathway upstream components, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor. In an aspect, the present invention relates to a drug or pharmaceutical composition for promoting the degradation of a misfolded protein and an aggregate thereof that contains one or more compounds. The one or more compounds are selected from: plasminogen activation pathway components, a compound capable of directly activating plasminogen or indirectly activating plasminogen by activating plasminogen activation pathway upstream components,

a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.

2. The method, the use, the drug or the pharmaceutical composition according to item 1, wherein the plasminogen activation pathway component is selected from plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, micro-plasmin, delta-plasminogen, delta-plasmin, a plasminogen activator, tPA, and uPA.

3. The method, the use, the drug or the pharmaceutical composition according to item 1, wherein the antagonist of the fibrinolysis inhibitor is an inhibitor of PAI-1, a complement C1 inhibitor, $\alpha$2 antiplasmin or an $\alpha$2 macroglobulin, such as an antibody.

4. The method, the use, the drug or the pharmaceutical composition according to any one of items 1 to 3, wherein the compound promotes the degradation of one or more misfolded proteins and aggregates thereof selected from: human amyloid A$\beta$40, human amyloid A$\beta$42, $\alpha$-synuclein, a Tau protein, an SOD-1 protein, polyglutamine, neuroserpin (NSP), a cystic fibrosis transmembrane conductance regulator, $\alpha$1-antitrypsin, a Parkin protein, crystallin, transthyretin, a short-chain acyl-CoA dehydrogenase (SCAD) variant, a low density lipoprotein receptor, huntingtin, a neurofilament protein, peripherin, an $\alpha$-internexin, islet amyloid polypeptide, $\beta$2-microglobulin, a serum amyloid A protein, an immunoglobulin light chain, human lysozyme, $\alpha$-lactalbumin, prothymosin $\alpha$, apolipoprotein E and apolipoprotein J.

In some embodiments, the compound promotes degradation of one or more proteins in brain tissues, the one or more proteins being selected from: the human amyloid A$\beta$40, the human amyloid A$\beta$42, the $\alpha$-synuclein, the Tau protein, the SOD-1 protein and the polyglutamine.

5. The method, the use, the drug or the pharmaceutical composition according to any one of items 1 to 3, wherein the compound promotes cleavage of Pro-BDNF to mature BDNF, or promotes cleavage of Pro-NGF to mature NGF. In some embodiments, the compound promotes cleavage of Pro-BDNF in the brain tissues to mature BDNF or promotes cleavage of Pro-NGF in the brain tissues to mature NGF.

6. The method, the use, the drug or the pharmaceutical composition according to any one of items 1 to 5, wherein the compound is plasminogen.

7. The method, the use, the drug or the pharmaceutical composition according to any one of items 1 to 6, wherein the plasminogen is human full-length plasminogen or a conservatively substituted variant thereof.

8. The method, the use, the drug or the pharmaceutical composition according to any one of items 1 to 6, wherein the plasminogen has at least 75%, 80%, 85%, 90%, 95%, 96%, 97%, 98% or 99% sequence identity with sequence 2 and still has the lysine binding activity or the proteolytic activity of plasminogen.

9. The method, the use, the drug or the pharmaceutical composition according to any one of items 1 to 6, wherein the plasminogen is a protein containing an amino acid sequence that has at least 80%, 90%, 95%, 96%, 97%, 98% or 99% amino acid sequence identity with sequence 14 and still having the proteolytic activity of plasminogen.

10. The method, the use, the drug or the pharmaceutical composition according to any one of items 1 to 6, wherein the plasminogen is selected from Glu-plasminogen, Lys-plasminogen, mini-plasminogen, micro-plasminogen, delta-plasminogen, and variants thereof that retain the proteolytic activity of plasminogen.

11. The method, the use, the drug or the pharmaceutical composition according to any one of items 1 to 6, wherein the plasminogen contains an amino acid sequence shown as sequence 2, 6, 8, 10 or 12, or contains a conservatively substituted variant of the amino acid sequence shown as sequence 2, 6, 8, 10 or 12.

12. The method, the use, the drug or the pharmaceutical composition according to any one of items 1 to 11, wherein the compound is used in combination with one or more other treatment methods or drugs.

13. The method, the use, the drug or the pharmaceutical composition according to item 12, wherein the other treatment methods include a cell therapy (including a stem cell therapy), a support therapy, and a physical therapy.

14. The method, the use, the drug or the pharmaceutical composition according to item 12, wherein the one or more other drugs are drugs for treating the Alzheimer's disease, the amyotrophic lateral sclerosis (ALS) or the Parkinson's disease.

15. The method, the use, the drug or the pharmaceutical composition according to any one of items 1 to 14, wherein the compound is administered by nasal inhalation, aerosol inhalation, nasal drops, eye drops, ear drops, an intravenous method, an intraperitoneal method, a subcutaneous method, an intracranial method, an intrathecal method, an intraarterial method (e.g. via the carotid artery) or an intramuscular method.

In addition, the present invention further relates to:

1. A method for preventing or treating diseases caused by a misfolded protein (which may also be collectively referred to as the misfolded protein diseases), including: administering a therapeutically effective amount of one or more compounds to a subject, the one or more compounds being selected from: plasminogen activation pathway components, a compound capable of directly activating plasminogen or indirectly activating plasminogen by activating plasminogen activation pathway upstream components , a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.

2. The method according to item 1, wherein the plasminogen activation pathway component is selected from plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, micro-plasmin, delta-plasminogen, delta-plasmin, a plasminogen activator, tPA and uPA.

3. The method according to item 1, wherein the antagonist of the fibrinolysis inhibitor is an inhibitor of PAI-1, a complement C1 inhibitor, $\alpha2$ antiplasmin or an $\alpha2$ macroglobulin, such as an antibody.

4. The method according to any one of items 1 to 3, wherein the diseases caused by a misfolded protein (which may also be collectively referred to as the misfolded protein diseases) include the Alzheimer's disease, the Parkinson's disease, amyotrophic lateral sclerosis and polyglutamine diseases, Gerstmann syndrome, fatal sporadic or familial isomnia, Kuru, cystic fibrosis, a liver injury, cataracts, familial amyloidosis, short-chain acyl-CoA dehydrogenase (SCAD) deficiency, familial hypercholesterolemia, a motor neuron disease, diabetes, dialysis-related amyloidosis and amyloidosis-related human diseases.

5. The method according to item 4, wherein the polyglutamine diseases include the Huntington's disease, spinocerebellar ataxia type 3 and dentatorubral-pallidoluysian atrophy.

6. The method according to any one of items 1 to 5, wherein the compound is plasminogen.

7. The method according to any one of items 1 to 6, wherein the plasminogen is human full-length plasminogen or a conservatively substituted variant thereof.

8. The method according to any one of items 1 to 6, wherein the plasminogen has at least 75%, 80%, 85%, 90%, 95%, 96%, 97%, 98% or 99% sequence identity with sequence 2 and still has the lysine binding activity or the proteolytic activity of plasminogen.

9. The method according to any one of items 1 to 6, wherein the plasminogen is a protein containing an amino acid sequence that has at least 80%, 90%, 95%, 96%, 97%, 98% or 99% amino acid sequence identity with sequence 14 and still having the proteolytic activity of plasminogen.

10. The method according to any one of items 1 to 6, wherein the plasminogen is selected from Glu-plasminogen, Lys-plasminogen, mini-plasminogen, micro-plasminogen, delta-plasminogen, and variants thereof that retain the proteolytic activity of plasminogen.

11. The method according to any one of items 1 to 6, wherein the plasminogen contains an amino acid sequence shown as sequence 2, 6, 8, 10 or 12, or contains a conservatively substituted variant of the amino acid sequences shown as sequence 2, 6, 8, 10 or 12.

12. The method according to any one of items 1 to 11, wherein the compound is used in combination with one or more other treatment methods or drugs.

13. The method according to item 12, wherein the other treatment methods comprise a cell therapy (comprising a stem cell therapy), a support therapy, and a physical therapy.

14. The method according to item 12, wherein the other drugs are other drugs for treating the Alzheimer's disease, the Parkinson's disease, the ALS and the Huntington's disease.

15. The method according to any one of items 1 to 14, wherein the compound is administered by nasal inhalation, aerosol inhalation, nasal drops, eye drops, ear drops, an intravenous method, an intraperitoneal method, a subcutaneous method, an intracranial method, an intrathecal method, an intraarterial method (e.g. via the carotid artery) or an intramuscular method.

In any one of the above embodiments of the present invention, the plasminogen may have at least 75%, 80%, 85%, 90%, 95%, 96%, 97%, 98% or 99% sequence identity with sequence 2, 6, 8, 10 or 12, and still have the activity, such as the lysine binding activity and the proteolytic activity, of plasminogen. In some embodiments, the plasminogen is a protein that is obtained by adding, deleting and/or substituting 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid on the basis of sequence 2, 6, 8, 10 or 12, and still has the activity, such as the lysine binding activity and the proteolytic activity, of plasminogen.

In some embodiments, the plasminogen is a protein containing an active plasminogen fragment and still having the activity, such as the proteolytic activity, of plasminogen. In some embodiments, the plasminogen is selected from Glu-plasminogen, Lys-plasminogen, mini-plasminogen, micro-plasminogen, delta-plasminogen, and variants thereof that retain the activity, such as the lysine binding activity and the proteolytic activity, of plasminogen. In some embodiments, the plasminogen is natural or synthesized human plasminogen, or a variant or fragment thereof that still retains the activity, such as the lysine binding activity and the proteolytic activity, of plasminogen. In some embodiments, the plasminogen is a human plasminogen ortholog from a primate or a rodent, or a variant or fragment thereof that retains the activity, such as the lysine binding activity and the proteolytic activity, of plasminogen. In some embodiments, the plasminogen has an amino acid sequence shown as sequence 2, 6, 8, 10 or 12. In some embodiments, the plasminogen is human natural plasminogen.

In some embodiments, the subject is a human. In some embodiments, the subject has the plasminogen deficiency. In some embodiments, the deficiency is congenital, secondary and/or local.

In some embodiments, the pharmaceutical composition contains a pharmaceutically acceptable carrier and plasminogen used in the above method. In some embodiments, the kit may be a preventative or therapeutic kit, which includes: (i) plasminogen used in the above method and (ii) a means for delivering the plasminogen to the subject. In some implementation, the means is a syringe or a vial. In some embodiments, the kit also contains a label or instructions. The label or the instructions indicate that the plasminogen is administered to the subject to implement any one of the above methods.

In some embodiments, the product contains: a container with a label, and (i) plasminogen used in the above method or a pharmaceutical composition containing plasminogen. The label indicates that the plasminogen or the composition is administered to the subject to implement any one of the above methods.

In some embodiments, the kit or the product also contains other one or more means or containers. The means or the containers contain other drugs.

In some embodiments of the above method, the plasminogen is administered systemically or locally, and preferably, the plasminogen is administered intravenously, intramuscularly or subcutaneously to treat the subject. In some embodiments of the above method, the plasminogen is administered in combination with a suitable polypeptide carrier or stabilizer. In some embodiments of the above method, the plasminogen is daily administered at a dose of 0.0001-2000 mg/kg, 0.001-800 mg/kg, 0.01-600 mg/kg, 0.1-400 mg/kg, 1-200 mg/kg, 1-100 mg/kg or 10-100 mg/kg (based on per kilogram of body weight), or is daily administered at a dose of 0.0001-2000 $mg/cm^2$, 0.001-800 $mg/cm^2$, 0.01-600 $mg/cm^2$, 0.1-400 $mg/cm^2$, 1-200 $mg/cm^2$, 1-100 $mg/cm^2$ or 10-100 $mg/cm^2$ (based on per square centimetre of body surface area), preferably, administration is repeated at least once, and preferably, administration is performed at least daily.

The present invention explicitly covers all combinations of the technical features belonging to the embodiments of the present invention, and the combined technical solutions have been explicitly disclosed in the present invention, just as the above technical solutions have been separately and explicitly disclosed. In addition, the present invention also explicitly covers combinations of the embodiments and their elements, and the combined technical solutions are explicitly disclosed herein.

BRIEF DESCRIPTION OF THE DRAWINGS

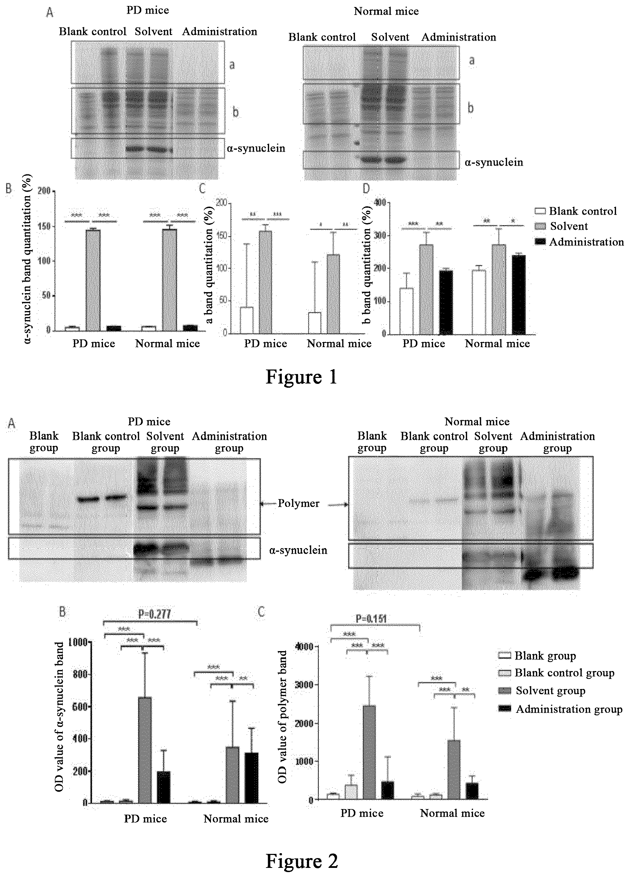

FIGS. 1A-D are diagrams showing results of effects of plasminogen on recombinant human $\alpha$-synuclein in cerebral homogenates of mice. A is a Tricine-SDS-PAGE electrophoretogram, and B, C and D are diagrams showing band scanning quantitative analysis results of $\alpha$-synuclein, an aggregate a and an aggregate b. The results show that in cerebral homogenates of mouse models of Parkinson's disease, the amount of $\alpha$-synuclein in a plasminogen group is significantly lower than that in a solvent control group (*** indicates P<0.001), and the amounts of the aggregates a and b in the plasminogen group are both significantly lower than those in the solvent control group (** indicates P<0.01, *** indicates P<0.001); and in cerebral homogenates of normal mice, the amount of the $\alpha$-synuclein in the plasminogen group is significantly lower than that in the solvent control group (*** indicates P<0.001), and the amounts of the aggregates a and b are significantly lower than those in the solvent control group (* indicates P<0.05, ** indicates P<0.01). It is indicated that in the cerebral homogenates of the mouse models of Parkinson's disease and the normal mice, the plasminogen may effectively promote the degradation of recombinant human $\alpha$-synuclein and aggregates thereof.

FIGS. 2A-C are diagrams showing results of effects of plasminogen on recombinant human $\alpha$-synuclein in cerebral homogenates of mice. A is a Western-blotting diagram; and B and C are diagrams showing band scanning quantitative analysis results of $\alpha$-synuclein and an aggregate thereof. The results show that in the cerebral homogenates of mouse models of Parkinson's disease, the amount of $\alpha$-synuclein in a plasminogen group is significantly lower than that in a solvent control group (** indicates P<0.01), and the amount of the aggregate of $\alpha$-synuclein is significantly lower than that in the solvent control group (*** indicates P<0.001); and in cerebral homogenates of normal mice, the amount of $\alpha$-synuclein in the plasminogen group is significantly lower than that in the solvent control group (** indicates P<0.01), and the amount of the aggregate of $\alpha$-synuclein is significantly lower than that in the solvent control group (*** indicates P<0.001). It is indicated that in the cerebral homogenates of the mouse models of Parkinson's disease and the normal mice, the plasminogen may effectively promote the degrada-

tion of recombinant human $\alpha$-synuclein and aggregates thereof.

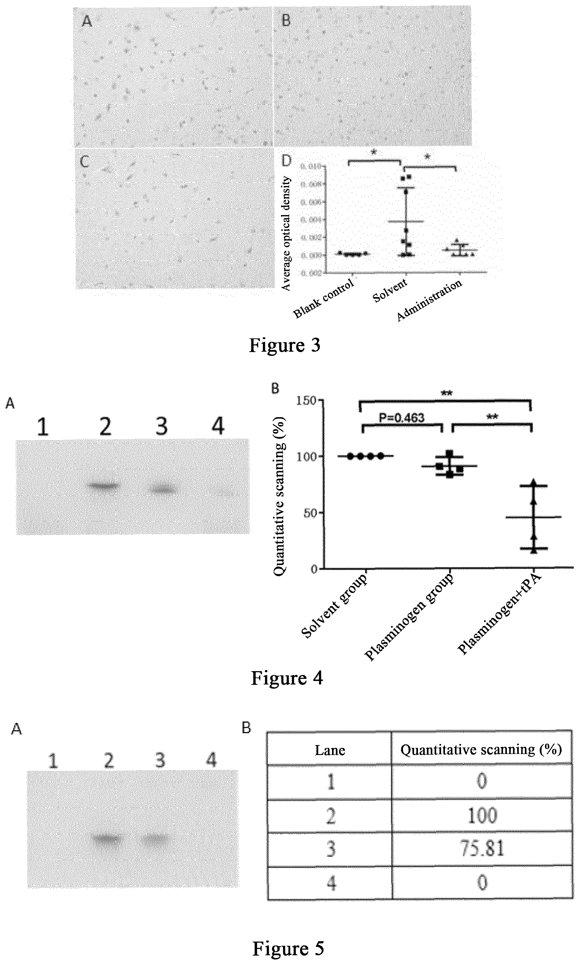

FIGS. 3A-D are diagrams showing immunohistochemical results for $\alpha$-synuclein in substantia nigra of mouse models of Parkinson's disease 14 days after plasminogen administration. A is a diagram showing a blank control group, B is a diagram showing a solvent group, C is a diagram showing a plasminogen group, and D is a diagram showing a quantitative analysis result of an average optical density. The results show that only a small amount of $\alpha$-synuclein is found in the substantia nigra of mice of the control group; the amount of $\alpha$-synuclein in the substantia nigra of mice in the solvent group is significantly higher than that in the blank control group (* indicates P<0.05); the amount of $\alpha$-synuclein in the substantia nigra of mice in the plasminogen group is significantly lower than that in the solvent group, and the statistical difference is significant (* indicates P<0.05); and the amount of $\alpha$-synuclein in the substantia nigra of mice in the plasminogen group approaches that in the blank control group. It is indicated that the plasminogen may reduce the expression level of $\alpha$-synuclein in the substantia nigra of the mouse models of Parkinson's disease and improve degeneration of a nerve injury.

FIGS. 4A-B are diagrams showing results of effects of plasminogen on human amyloid A$\beta$40 in PBS buffer systems. A is a Tricine-SDS-PAGE electrophoretogram, and B is a diagram showing a quantitative scanning analysis result of in vitro dissolution of A$\beta$40. The results show that the amount of A$\beta$40 in a solvent control group is defined as 100% and does not have any change; A$\beta$40 in a plasminogen group is partially degraded in a case that plasminogen is added alone; and A$\beta$40 in a plasminogen+tPA group is significantly degraded in vitro in a case that plasminogen and tPA are added, and the difference between the plasminogen+tPA group and the solvent control group is significant (** indicates P<0.01). It is indicated that the plasminogen may promote the degradation of human amyloid A$\beta$40 in the PBS buffer system.

FIGS. 5A-B are diagrams showing results of effects of plasminogen on human amyloid A$\beta$40 in cerebrospinal fluids of rabbits. A is a Tricine-SDS-PAGE electrophoretogram, and B is a diagram showing a quantitative scanning analysis result of dissolution of A$\beta$40. The results show that the amount of A$\beta$40 in a solvent control group is defined as 100% and does not have any change; and A$\beta$40 in a plasminogen group is partially degraded in a case that plasminogen is added alone and is degraded to 74.81%. It is indicated that the plasminogen may promote the degradation of human A$\beta$40 in the cerebrospinal fluids of the rabbits.

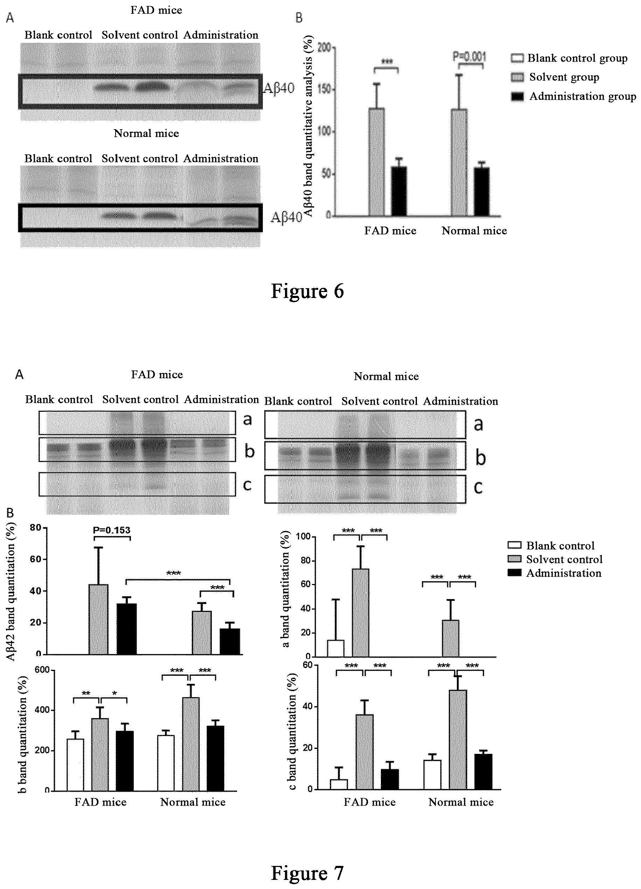

FIGS. 6A-B are diagrams showing results of effects of plasminogen on human A$\beta$40 in cerebral homoge-

nates of mouse models of Alzheimer's disease and normal mice. A is a Tricine-SDS-PAGE electrophoretogram, and B is a diagram showing a quantitative scanning analysis result of in vitro dissolution of Aβ40. The results show that in the cerebral homogenates of the mouse models of Alzheimer's disease, the amount of human amyloid Aβ40 of a plasminogen group is significantly less than that of a solvent control group, and the difference is extremely significant (*** indicates P<0.001); and in the cerebral homogenates of the normal mice, the amount of amyloid Aβ40 of the plasminogen group is significantly less than that of the solvent control group, and the difference is extremely significant (P=0.001). It is indicated that plasminogen may effectively promote the degradation of human Aβ40 in the cerebral homogenates of the mouse models of Alzheimer's disease and the normal mice.

FIGS. 7A-B are diagrams showing results of effects of plasminogen on human Aβ42 in cerebral homogenates of mouse models of Alzheimer's disease and normal mice. A is a Tricine-SDS-PAGE electrophoretogram, and B is a diagram showing quantitative scanning analysis results of in vitro dissolution of Aβ42 and aggregates thereof. The results show that in the cerebral homogenates of the mouse models of Alzheimer's disease, the amount of amyloid Aβ42 of a plasminogen group is less than that of a solvent control group, and the amounts of aggregates a, b, and c of the plasminogen group are all less than those of the solvent group, and the difference is extremely significant (* indicates P<0.05, and *** indicates P<0.001); and in the cerebral homogenates of the normal mice, the amount of amyloid Aβ42 of the plasminogen group is obviously less than that of the solvent control group, the difference is extremely significant (*** indicates P<0.001), the amounts of aggregates a, b, and c are all less than those of the solvent group, and the difference is extremely significant (*** indicates P<0.001). It is indicated that plasminogen may effectively promote the degradation of human Aβ42 in the cerebral homogenates of the mouse models of Alzheimer's disease and the normal mice.

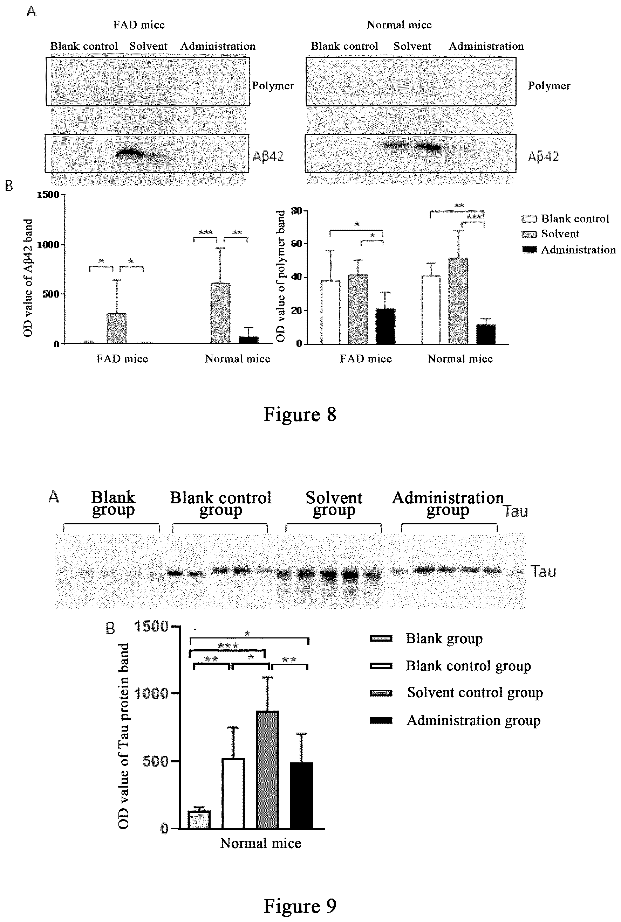

FIGS. 8A-B are diagrams showing results of effects of plasminogen on human Aβ42 in cerebral homogenates of mouse models of Alzheimer's disease and normal mice. A is a Western-blotting diagram, and B is a diagram showing quantitative scanning analysis results of in vitro dissolution of Aβ42 and aggregates thereof. The results show that in the cerebral homogenates of the mouse models of Alzheimer's disease, the amount of amyloid Aβ42 in a plasminogen group is less than that of a solvent control group, and the amounts of the aggregates of the plasminogen group are all significantly less than those of the solvent group, and the difference is extremely significant (* indicates P<0.05); and in the cerebral homogenates of the normal mice, the amount of amyloid Aβ42 of the plasminogen group is significantly less than that of the solvent control group, the difference is extremely significant (** indicates P<0.01), the amounts of aggregates are all less than those of the solvent group, and the difference is extremely significant (*** indicates P<0.001). It is indicated that plasminogen may effectively promote the degradation of human amyloid Aβ42 and aggregates thereof in the cerebral homogenates of the mouse models of Alzheimer's disease and the normal mice.

FIGS. 9A-B are diagrams showing results of effects of plasminogen on Tau proteins in cerebral homogenates of normal mice. A is a Western blot image, and B is a diagram showing quantitative analysis results of optical densities of Tau protein bands. The results show that in the cerebral homogenates of the normal mice, the amount of Tau proteins of a plasminogen group is significantly less than that of a solvent control group, and the difference is significant (* indicates P<0.05, ** indicates P<0.01, and *** indicates P<0.001). It is indicated that the plasminogen may promote the degradation of Tau proteins in the cerebral homogenates of the normal mice.

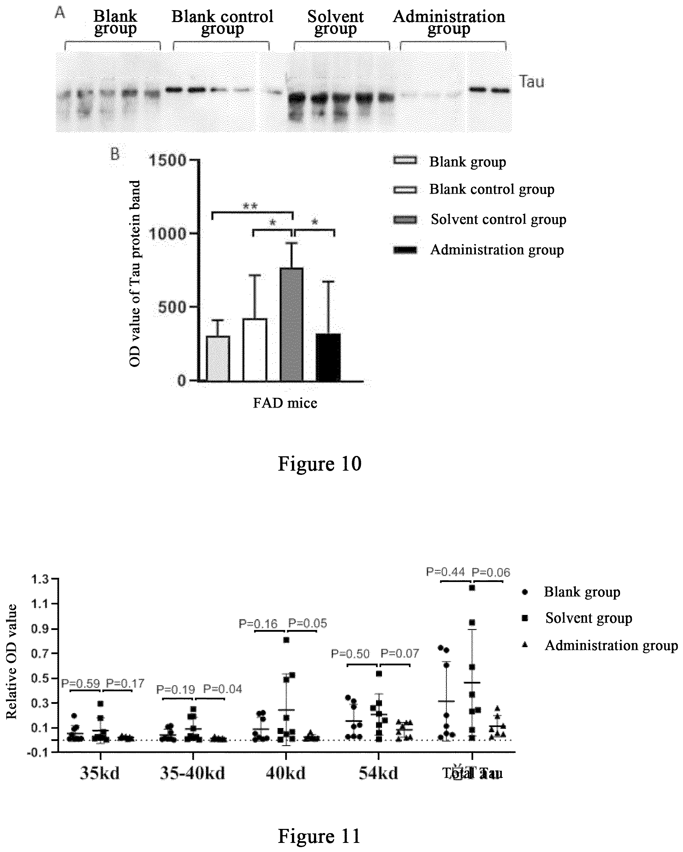

FIGS. 10A-B are diagrams showing results of effects of plasminogen on Tau proteins in cerebral homogenates of mouse models of Alzheimer's disease. A is a Western blot image, and B is a diagram showing quantitative analysis results of optical densities of Tau protein bands. The results show that in the cerebral homogenates of the mouse models of Alzheimer's disease, the amount of Tau proteins of a plasminogen group is significantly less than that of a solvent control group, and the statistical difference is significant (* indicates P<0.05, and ** indicates P<0.01). It is indicated that the plasminogen may promote the degradation of Tau proteins in the cerebral homogenates of the mouse models of Alzheimer's disease.

FIG. 11 is a diagram showing Western blot detection results of Tau proteins having different molecular weights in brain tissues of Alzheimer's mice after 28 days of plasminogen administration. The results show that certain levels of Tau proteins having different molecular weights are present in cerebral homogenates of mice in a blank control group; the levels of Tau proteins having different molecular weights and the total Tau protein level in brain tissues of mice in an administration group are significantly lower than those in a solvent group, and statistical analysis P values between the two groups in the levels of Tau proteins having molecular weights of 35 kd, 35-40 kd, 40 kd, and 54 kd, and the total Tau protein level are 0.174, 0.0406, 0.052, 0.067, and 0.055, respectively. It is indicated that the plasminogen can promote the degradation of Tau proteins in brain tissues of the mouse models of Alzheimer's disease.

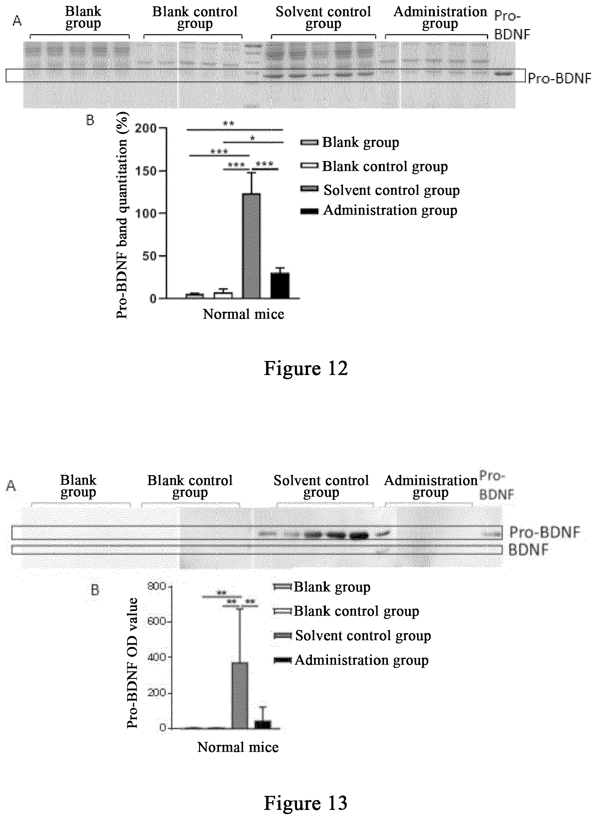

FIGS. 12A-B are diagrams showing results of effects of plasminogen on recombinant human Pro-BDNF in cerebral homogenates of normal mice. A is an SDS-PAGE electrophoregram, and B is a diagram showing an SDS-PAGE band quantitative analysis result. The results show that in the cerebral homogenates of the normal mice, the amount of Pro-BDNF in a plasminogen administration group is significantly lower than that in a solvent control group, and the difference is extremely significant (*** indicates P<0.001). It is suggested that plasminogen can promote cleavage of recombinant human Pro-BDNF in the cerebral homogenates of the normal mice.

FIGS. 13A-B are diagrams showing results of effects of plasminogen on recombinant human Pro-BDNF in cerebral homogenates of normal mice. A is a Western blot image; and B is a diagram showing an analysis result of an optical density (OD) value of a Pro-BDNF band in Western blot. The results show that in the cerebral homogenates of the normal mice, the amount of Pro-BDNF in a plasminogen administration group is significantly lower than that in a solvent control group, and the difference is extremely significant (** indicates P<0.01). It is suggested that plasminogen can promote cleavage of recombinant human Pro-BDNF in the cerebral homogenates of the normal mice.

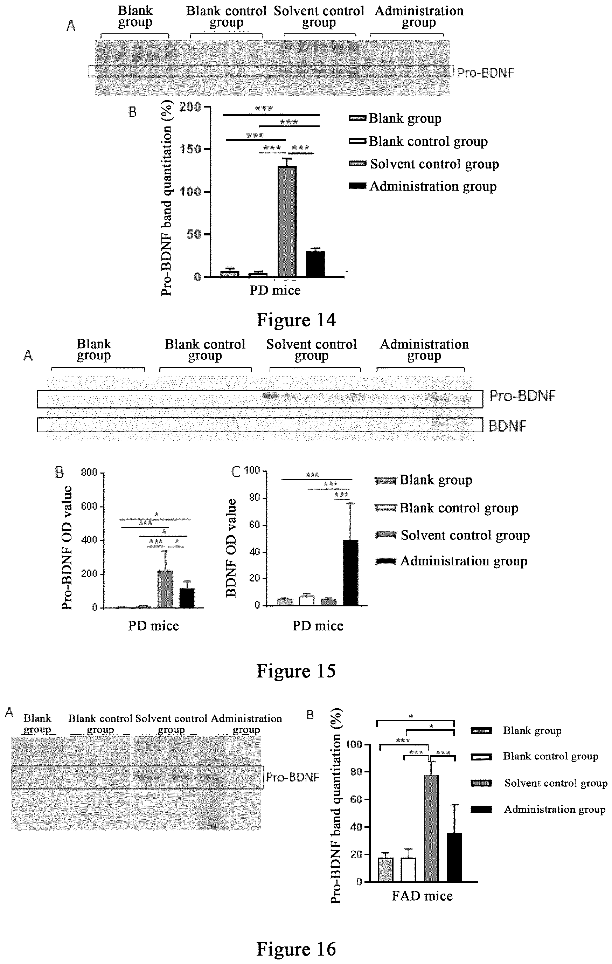

FIGS. 14A-B are diagrams showing results of effects of plasminogen on recombinant human Pro-BDNF in cerebral homogenates of mouse models of Parkinson's disease. A is an SDS-PAGE image, and B is a diagram showing an SDS-PAGE band quantitative analysis result. The results show that in the cerebral homogenates of the mouse models of Parkinson's disease, the amount of Pro-BDNF in a plasminogen administration group is significantly less than that in a solvent control group, and the difference is extremely significant (*** indicates P<0.001). It is suggested that plasminogen can promote cleavage of recombinant human Pro-BDNF in the cerebral homogenates of the mouse models of Parkinson's disease.

FIGS. 15A-C are diagrams showing effects of plasminogen on recombinant human Pro-BDNF in cerebral homogenates of mouse models of Parkinson's disease. A shows a Western blot image, B shows analysis results of optical density (OD) values of Pro-BDNF bands in the Western blot image, and C shows analysis results of optical density (OD) values of BDNF bands in the Western blot image. The results show that in the cerebral homogenates of the mouse models of Parkinson's disease, the amount of Pro-BDNF in a plasminogen administration group is significantly lower than that in a solvent control group, and the difference is significant (* indicates P<0.05, *** indicates P<0.001); and the amount of BDNF in the plasminogen administration group is significantly higher than that in the solvent control group, and the difference is very significant. It is suggested that plasminogen can promote cleavage of recombinant human Pro-BDNF and formation of mature BDNF in the cerebral homogenates of the mouse models of the Parkinson's disease.

FIGS. 16A-B are diagrams showing effects of plasminogen on recombinant human Pro-BDNF in cerebral homogenates of mouse models of Alzheimer's disease. A is an SDS-PAGE electrophoregram, and B is a diagram showing quantitative analysis results of Pro-BDNF bands in the SDS-PAGE electrophoregram. The results show that in the cerebral homogenates of the mouse models of Alzheimer's disease, the amount of Pro-BDNF in a plasminogen administration group is significantly less than that in a solvent control group, and the difference is extremely significant (*** indicates P<0.001). It is indicated that plasminogen can promote the cleavage of Pro-BDNF in the cerebral homogenates of the mouse models of Alzheimer's disease.

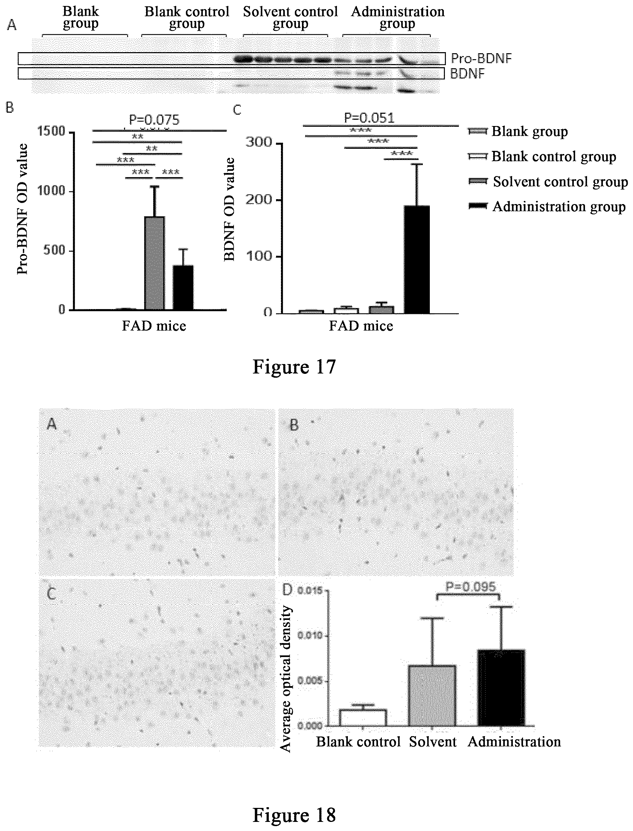

FIGS. 17A-C are diagrams showing effects of plasminogen on recombinant human Pro-BDNF in cerebral homogenates of mouse models of Alzheimer's disease, wherein A is a Western blot image, B is a diagram showing analysis results of optical density (OD) values of Pro-BDNF bands in the Western blot image, and C is a diagram showing analysis results of optical density (OD) values of BDNF bands in the Western blot image. The results show that in the cerebral homogenates of the mouse models of Alzheimer's disease, the amount of Pro-BDNF in a plasminogen administration group is significantly less than that in a solvent control group, and the difference is extremely significant (** indicates P<0.01, and *** indicates P<0.001); the amount of BDNF content in the plasminogen administration group is significantly greater than that in the solvent control group, and the difference is extremely significant. It is indicated that plasminogen can promote the cleavage of Pro-BDNF and formation of mature BDNF in the cerebral homogenates of the mouse models of Alzheimer's disease.

FIGS. 18A-D are diagrams showing immunohistochemical staining results of BDNF in the hippocampus of mouse models of schizophrenia 35 days after plasminogen administration. A shows a blank control group, B shows a solvent PBS control group, C shows a plasminogen administration group, and D shows quantitative analysis results of the average optical density. The results show that there is a certain level of BDNF (indicated by an arrow) in the hippocampus of mice in the blank control group; the level of BDNF in the hippocampus of mice in the solvent group is increased; the level of BDNF in the hippocampus of mice in the administration group is significantly higher than that in the solvent group, and the statistical difference is close to significance (P=0.095). It is suggested that the plasminogen can increase the level of BDNF in the hippocampus of the mouse models of schizophrenia.

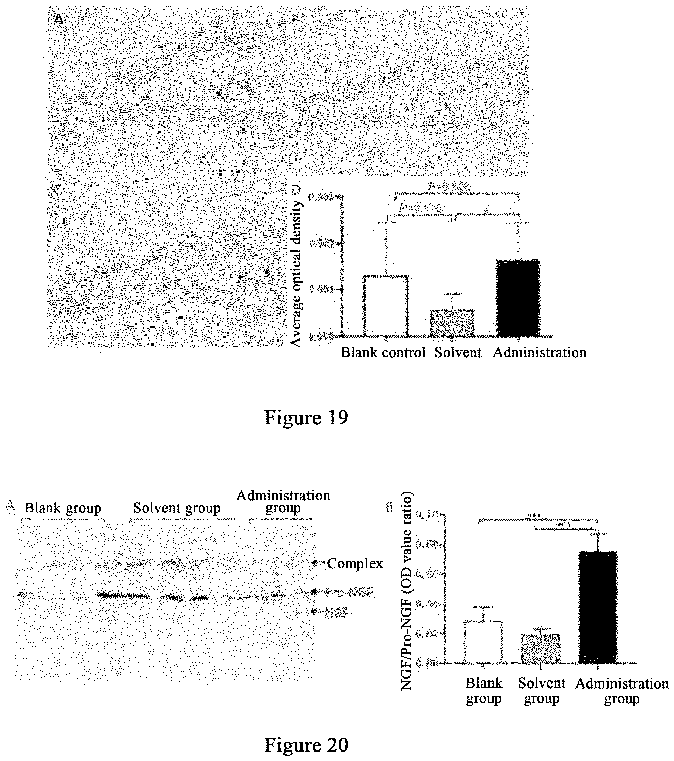

FIGS. 19A-D are diagrams showing immumohistochemical staining results of BDNF in the hippocampus of mouse models of Alzheimer's disease after 28 days of plasminogen administration. A shows a blank control group, B shows a solvent group, C shows an administration group, and D shows quantitative analysis results of the average optical density. The results show that a certain level of BDNF (indicated by arrows) is expressed in the hippocampus of mice in the blank control group; the expression of BDNF in the hippocampus of mice in the solvent group is significantly lower than that of mice in the blank control group; the expression of BDNF in the hippocampus of mice in the administration group is significantly greater than that of mice in the solvent group, and the statistical difference is significant (* indicates $P<0.05$). It is indicated that plasminogen can promote the expression of BDNF in the hippocampus of the mouse models of Alzheimer's disease.

FIGS. 20A-B are diagrams showing results of Western blot tests and quantitative analysis results of NGF/Pro-NGF optical density (OD) ratios of representative brain tissues in SMA mice after plasminogen administration. The results show that there is a certain NGF/ProNGF ratio in the brain tissues of mice in a blank control group, the NGF/ProNGF ratio in the brain tissues of the mice in administration group is significantly higher than that of mice in a solvent group, and the statistical difference is very significant (*** indicates $P<0.001$). It is suggested that plasminogen can promote transformation of ProNGF into NGF and formation of mature NGF in the brain tissues of SMA mouse models.

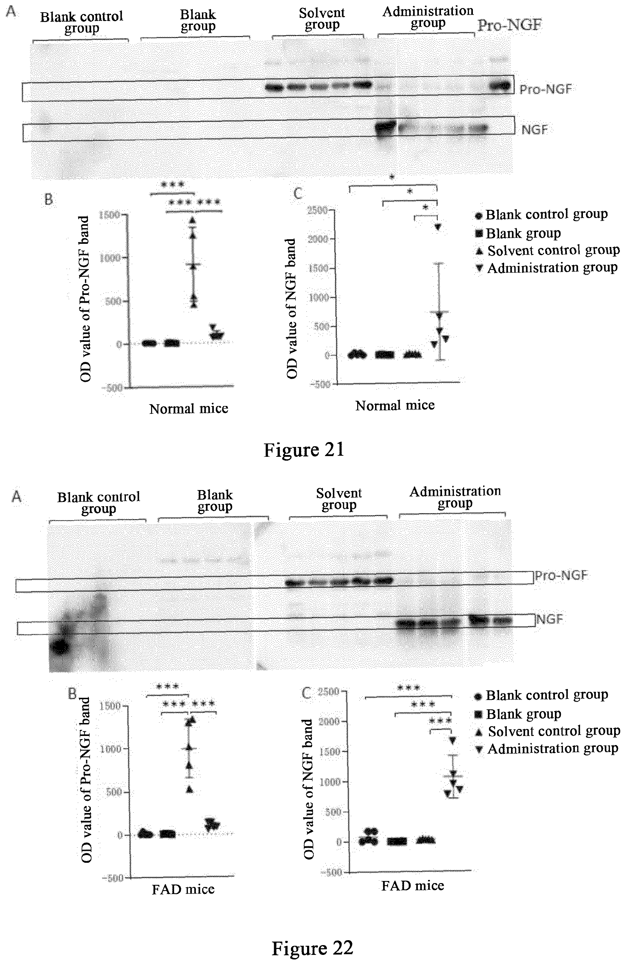

FIGS. 21A-C are diagrams showing effects of plasminogen on recombinant human Pro-NGF in cerebral homogenates of normal mice, wherein A shows a Western blot image, B shows analysis results of optical density (OD) values of Pro-NGF bands in the Western blot image, and C shows analysis results of optical density (OD) values of NGF bands in the Western blot image. The results show that in the cerebral homogenates of the normal mice, the amount of Pro-NGF in a plasminogen administration group is significantly less than that in a solvent control group, and the difference is extremely significant (* indicates $P<0.05$, and *** indicates $P<0.001$); the amount of NGF content in the plasminogen administration group is significantly greater than that in the solvent control group, and the difference is extremely significant. It is suggested that plasminogen can promote cleavage of recombinant human Pro-NGF and formation of mature NGF in the cerebral homogenates of the normal mice.

FIGS. 22A-C are diagrams showing effects of plasminogen on recombinant human Pro-NGF in cerebral homogenates of mouse models of Alzheimer's disease. A shows a Western blot image, B shows analysis results of optical density (OD) values of Pro-NGF bands in the Western blot image, and C shows analysis results of optical density (OD) values of NGF bands in the Western blot image. The results show that in the cerebral homogenates of the mouse models of Alzheimer's disease, the amount of Pro-NGF content in a plasminogen administration group is significantly less than that in a solvent control group, and the difference is extremely significant (*** indicates $P<0.001$); the amount of NGF in the plasminogen administration group is significantly greater than that in the solvent control group, and the difference is significant. It is indicated that plasminogen can promote the cleavage of recombinant human Pro-NGF and formation of mature NGF in the cerebral homogenates of the mouse models of Alzheimer's disease.

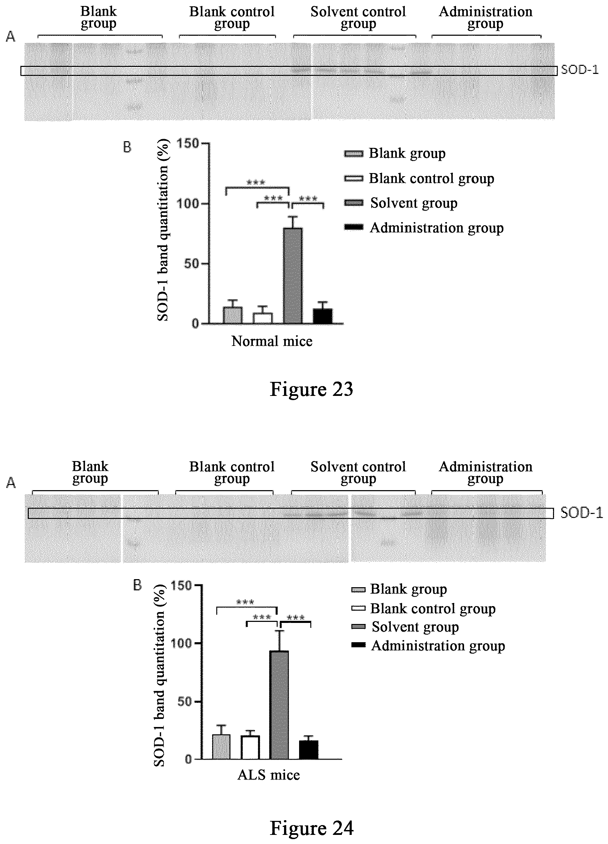

FIGS. 23A-B are diagrams showing effects of plasminogen on the recombinant SOD-1 protein in cerebral homogenates of normal mice. A is an SDS-PAGE electrophoretogram; and B is a diagram showing a quantitative scanning analysis result of in vitro dissolution of the SOD-1 protein. The results show that in the cerebral homogenates of the normal mice, the amount of SOD-1 in a plasminogen group is significantly lower than that in a solvent control group, and the difference is significant (*** indicates $P<0.001$). It is indicated that the plasminogen may effectively promote the degradation of the SOD-1 protein in the cerebral homogenates of the normal mice.

FIGS. 24A-B are diagrams showing results of effects of plasminogen on the recombinant SOD-1 protein in cerebral homogenates of SOD1-G93A transgenic mice. A is an SDS-PAGE electrophoretogram; and B is a diagram showing a quantitative scanning analysis result of in vitro dissolution of the SOD-1 protein. The results show that in the cerebral homogenates of the SOD1-G93A transgenic mice, the amount of the SOD-1 protein in a plasminogen group is significantly lower than that in a solvent control group, and the difference is very significant (*** indicates $P<0.001$). It is indicated that the plasminogen may effectively promote the degradation of the SOD-1 protein in cerebral homogenates of ALS model mice.

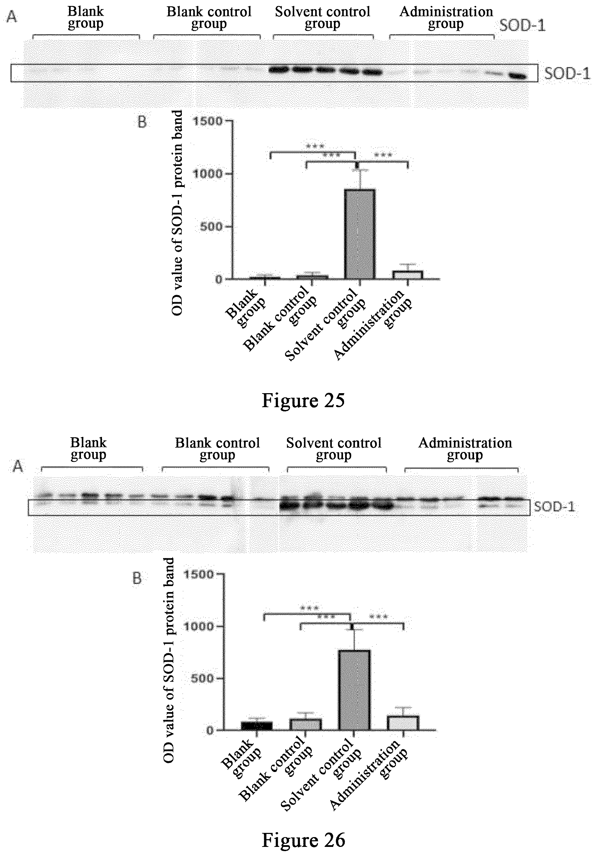

FIGS. 25A-B are diagrams showing effects of plasminogen on the recombinant SOD-1 protein in cerebral homogenates of normal mice. A is a Western blot image, and B is a diagram showing an analysis result of optical density (OD) values of SOD-1 protein bands in Western blot. The results show that in the cerebral homogenates of the normal mice, the amount of the SOD-1 protein in a plasminogen group is significantly lower than that in a solvent control group, and the difference is very significant (*** indicates $P<0.001$). It is indicated that the plasminogen may effectively promote the degradation of the SOD-1 protein in the cerebral homogenates of the normal mice.

FIGS. 26A-B are diagrams showing effects of plasminogen on the recombinant SOD-1 protein in cerebral homogenates of SOD1-G93A transgenic mice. A is a Western blot image, and B is a diagram showing an analysis result of optical density (OD) values of SOD-1 protein bands in Western blot. The results show that in the cerebral homogenates of the SOD1-G93A transgenic mice, the amount of the SOD-1 protein in a plasminogen group is significantly lower than that in a solvent control group, and the difference is very significant (*** indicates P<0.001). It is indicated that the plasminogen may effectively promote the degradation of the SOD-1 protein in the cerebral homogenates of the SOD1-G93A transgenic mice.

DETAILED DESCRIPTION OF THE INVENTION

"Protein folding" is the process that polypeptide chains are coiled to form specific functional three-dimensional structures or conformations simultaneously with or subsequent to their synthesis on ribosomes according to principles of thermodynamics and kinetics, or with the assistance of molecular chaperones. Correctly folded proteins have normal functions, while a misfolded protein may lead to diseases.

Fibrinolytic system is a system composed of a series of involved in fibrinolysis. The chemical substances mainly include plasminogen, plasmin, plasminogen activators, and fibrinolysis inhibitors. The plasminogen activators include a tissue-type plasminogen activator (t-PA) and a urokinase-type plasminogen activator (u-PA). t-PA is a serine protease synthesized by vascular endothelial cells, t-PA activates plasminogen mainly on fibrin. The urokinase-type plasminogen activator (u-PA) is produced by renal tubular epithelial cells and vascular endothelial cells, and can directly activate plasminogen without the need for fibrin as a cofactor. Plasminogen (PLG) is synthesized in the liver. When blood coagulates, a large amount of PLG is adsorbed onto the fibrin network, and is activated to plasmin under the action of t-PA or u-PA to promote fibrinolysis. Plasmin (PL) is a serine protease, and has the following effects: degrading fibrin and fibrinogen; hydrolyzing a variety of blood coagulation factors such as V, VIII, X, VII, XI, and II; enabling plasminogen to be transformed into plasmin; hydrolyzing complements, etc. The fibrinolysis inhibitors include: plasminogen activator inhibitors (PAIs) and α2 antiplasmin (α2-AP). PAIs mainly include two types, i.e. PAI-1 and PAI-2, and can specifically bind to t-PA in a ratio of 1: 1 to inactivate t-PA and activate PLG at the same time. α2-AP is synthesized in the liver, and binds to PL in a ratio of 1: 1 to form a complex so as to inhibit the activity of PL. F X III enables α2-AP to bind to fibrin in the form of covalent bond to attenuate the sensitivity of fibrin to the action of PL. Substances inhibiting the activity of the fibrinolytic system in vivo include: PAI-1, a complement C1 inhibitor, α2 antiplasmin, and an α2 macroglobulin.

Herein, the term "plasminogen activation pathway component" covers:

1. plasminogen, Lys-plasminogen, Glu-plasminogen, micro-plasminogen, delta-plasminogen, and variants and analogs thereof;
2. plasmin and variants and analogs thereof; and
3. plasminogen activators, such as tPA, uPA, and a tPA or uPA variant or analog containing one or more domains (e.g. one or more kringle domains and proteolysis domains) of tPA or uPA.

The above "variants" of plasminogen, plasmin, tPA, and uPA include all naturally occurring human genetic variants and other mammalian forms of these proteins, and proteins that are obtained by adding, deleting and/or substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid and still has the activity of plasminogen, plasmin, tPA or uPA. For example, the "variants" of plasminogen, plasmin, tPA, and uPA include mutational variants of these proteins that are obtained by substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid with conservative amino acids.

The "plasminogen variants" of the present invention include proteins having at least 75%, 80%, 85%, 90%, 95%, 96%, 97%, 98% or 99% sequence identity with sequence 2, 6, 8, 10 or 12 and still having the activity, such as the lysine binding activity and the proteolytic activity, of plasminogen. For example, the "plasminogen variants" of the present invention may be proteins that are obtained by adding, deleting and/or substituting 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid on the basis of sequence 2, 6, 8, 10 or 12 and still have the activity, such as the lysine binding activity and the proteolytic activity, of plasminogen. Specifically, the plasminogen variants of the present invention include all naturally occurring human genetic variants and other mammalian forms of these proteins, and mutational variants of these proteins that are obtained by substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid with conservative amino acids.

The plasminogen of the present invention may be a human plasminogen ortholog from a primate or a rodent, or a variant thereof that retains the activity, such as the lysine binding activity and the proteolytic activity, of plasminogen, such as plasminogen shown as sequence 2, 6, 8, 10 or 12, and human natural plasminogen shown as sequence 2.

The above "analogs" of plasminogen, plasmin, tPA, and uPA include compounds respectively providing functions basically similar to those of plasminogen, plasmin, tPA, or uPA.

The above "variants" and "analogs" of plasminogen, plasmin, tPA, and uPA include "variants" and "analogs" containing one or more domains (e.g. one or more kringle domains and proteolysis domains) of plasminogen, plasmin, tPA, and uPA. For example, the "variants" and "analogs" of plasminogen include plasminogen variants and analogs containing one or more domains (e.g. one or more kringle domains and proteolysis domains) of plasminogen, such as mini-plasminogen. The "variants" and "analogs" of plasmin include plasmin "variants" and "analogs" containing one or more domains (e.g. one or more kringle domains and proteolysis domains) of plasmin, such as mini-plasmin and delta-plasmin ($\delta$-plasmin).

Whether the above "variants" or "analogs" of plasminogen, plasmin, tPA or uPA have the activity of plasminogen, plasmin, tPA or uPA, or whether the above "variants" or "analogs" of plasminogen, plasmin, tPA or uPA respectively provide functions basically similar to those of plasminogen, plasmin, tPA or uPA can be detected by the methods known in the art. For example, the activity of activated plasmin is determined by enzymography, an enzyme-linked immunosorbent assay (ELISA) or fluorescence-activated cell sorting (FACS), or determined by the methods described in the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest. 74 (5): 1625-33; Gravanis I, Tsirka SE (February 2008). "Tissue-type plasminogen activator as a therapeutic target in stroke". Expert Opinion on Therapeutic Targets. 12 (2): 159-70; and Geiger M, Huber K, Wojta J, Stingl L, Espana F, Griffin JH, Binder BR (Aug 1989). "Complex formation between urokinase and plasma protein C inhibitor in vitro and in vivo". Blood. 74 (2): 722-8.

In some embodiments of the present invention, the "plasminogen activation pathway component" of the present invention is plasminogen. In some embodiments, the plasminogen is human full-length plasminogen or a conservatively substituted variant thereof that retains the activity (e.g. the lysine binding activity and the proteolytic activity) of plasminogen. In some embodiments, the plasminogen is selected from Glu-plasminogen, Lys-plasminogen, mini-plasminogen, micro-plasminogen, delta-plasminogen, and variants thereof that retain the activity (e.g. the lysine binding activity and the proteolytic activity) of plasminogen. In some embodiments, the plasminogen is natural or synthesized human plasminogen, or a conservatively substituted variant or fragment thereof that retains the activity (e.g. the lysine binding activity and the proteolytic activity) of plasminogen. In some embodiments, the plasminogen is a human plasminogen ortholog from a primate or a rodent, or a conservatively substituted variant or fragment thereof that retains the activity of plasminogen. In some embodiments, the plasminogen contains an amino acid sequence shown as sequence 2, 6, 8, 10 or 12. In some embodiments, the plasminogen contains a conservatively substituted sequence of the amino acid sequence shown as sequence 2, 6, 8, 10 or 12. In some embodiments, the plasminogen has an amino acid sequence shown as sequence 2, 6, 8, 10 or 12. In some embodiments, the plasminogen is a conservatively substituted variant of plasminogen shown as sequence 2, 6, 8, 10 or 12. In some embodiments, the plasminogen is human natural plasminogen or a conservative mutant thereof. In some embodiments, the plasminogen is human natural plasminogen shown as sequence 2 or a conservatively substituted variant thereof.

A "compound capable of directly activating plasminogen or indirectly activating plasminogen by activating an upstream component of a plasminogen activation pathway" refers to any compound that can directly activate plasminogen or indirectly activate plasminogen by activating an upstream component of a plasminogen activation pathway, such as tPA, uPA, streptokinase, saruplase, alteplase, reteplase, tenecteplase, anistreplase, monteplase, lanoteplase, pamiteplase, and staphylokinase.

An "antagonist of a fibrinolysis inhibitor" of the present invention is a compound that antagonizes, weakens, blocks, or prevents the action of a fibrinolysis inhibitor. The fibrinolysis inhibitor is, for example, PAI-1, a complement C1 inhibitor, $\alpha 2$ antiplasmin or an $\alpha 2$ macroglobulin. The antagonist is, for example, an antibody of PAI-1, a complement C1 inhibitor, $\alpha 2$ antiplasmin or an $\alpha 2$ macroglobulin, or antisense RNA or small RNA that blocks or down-regulates the expression of PAI-1, a complement C1 inhibitor, $\alpha 2$ antiplasmin or an $\alpha 2$ macroglobulin, or a compound that occupies a binding site of PAI-1, a complement C1 inhibitor, $\alpha 2$ antiplasmin or an $\alpha 2$ macroglobulin and does not have functions of PAI-1, a complement C1 inhibitor, $\alpha 2$ antiplasmin or an $\alpha 2$ macroglobulin, or compound that blocks a binding domain and/or an activity domain of PAI-1, a complement C1 inhibitor, $\alpha 2$ antiplasmin or an $\alpha 2$ macroglobulin.

Plasmin is a key component of a plasminogen activation system. It is a broad-spectrum protease, and can hydrolyze several components, including fibrin, gelatin, fibronectin, laminin, and proteoglycans, of an extracellular matrix (ECM). In addition, plasmin can activate some matrix metalloproteinase precursors (pro-MMPs) to active matrix metalloproteinases (MMPs) Therefore, plasmin is considered as an important upstream regulator of extracellular proteolysis. Plasmin is formed by proteolysis of plasminogen with two types of physiological PAs, i.e. a tissue-type plasminogen activator (tPA) and a urokinase-type plasminogen activator (uPA). Due to relatively high levels of plasminogen in plasma and other body fluids, it has traditionally been thought that the regulation of the PA system is mainly achieved through the synthesis and activity levels of PAs. The synthesis of components of the PA system is strictly regulated by different factors, such as a hormone, a growth factor, and a cytokine. In addition, there are specific physiological inhibitors of plasmin and PAs. A main inhibitor of plasmin is α2-antiplasmin. The activity of PAs is regulated by both of a plasminogen activator inhibitor 1 (PAI-1) for inhibiting uPA and tPA and a plasminogen activator inhibitor 2 (PAI-2) for mainly inhibiting uPA. There are uPA-specific cell surface receptors (uPARs) having the direct hydrolysis activity on the surface of some cells.

Plasminogen is a single-stranded glycoprotein, is composed of 791 amino acids, and has a molecular wight of about 92 kDa. Plasminogen is mainly synthesized in the liver, and is abundant in the extracellular fluid. The plasminogen content in plasma is about 2 μM. Therefore, plasminogen is a huge potential source of the proteolytic activity in tissues and body fluids. Plasminogen is present in two molecular forms, i.e. glutamate-plasminogen (Glu-plasminogen) and lysine-plasminogen (Lys-plasminogen). Naturally secreted and uncleaved plasminogen has an amino-terminal (N-terminal) glutamate, so it is referred to as glutamate-plasminogen. However, glutamate-plasminogen is hydrolyzed to lysine-plasminogen at Lys76-Lys77 in the presence of plasmin. Compared with glutamate-plasminogen, lysine-plasminogen has higher affinity to fibrin and can be activated by PAs at a higher rate. Arg560-Val561 peptide bonds of the two forms of plasminogen can be cleaved by uPA or tPA to form double-stranded protease plasmin linked via a disulfide bond. The amino-terminal moiety of plasminogen contains five homologous tri-circles, i.e. kringles; and the carboxyl-terminal moiety of plasminogen contains protease domains. Some kringles contain lysine binding sites for mediating specific interaction of plasminogen and fibrin, as well as its inhibitor α2-AP. It is found recently that a plasminogen fragment of 38 kDa that contains kringles 1 to 4 is an effective inhibitor for angiogenesis. This fragment is named angiostatin, which can be produced by hydrolyzing plasminogen with several proteases.

A main substrate of plasmin is fibrin, and the dissolution of fibrin is a key for preventing pathological thrombosis. Plasmin also has substrate specificity to several components, including laminin, fibronectin, proteoglycans, and gelatin, of ECM, suggesting that plasmin also plays an important role in reconstruction of ECM. Indirectly, plasmin can also degrade other components, including MMP-1, MMP-2, MMP-3, and MMP-9, of ECM by transforming some protease precursors into active proteases. Therefore, it has been proposed that plasmin may be an important upstream regulator of extracellular proteolysis. In addition, plasmin has the ability to activate certain potential growth factors. In vitro, plasmin can also hydrolyze components of a complement system and release chemotactic complement fragments.

"Plasmin" is a very important enzyme present in the blood, which can hydrolyze a fibrin clot to fibrin degradation products and D-dimer.

"Plasminogen" is the zymogen form of plasmin. According to sequences in Swiss Prot, a glycoprotein composed 810 amino acids, having a molecular weight of about 90 kDa, mainly synthesized in the liver, and capable of circulating in the blood is calculated based on an amino acid sequence (sequence 4) of natural human plasminogen containing a signal peptide, and a cDNA sequence for encoding the amino acid sequence is shown as sequence 3. Full-length plasminogen contains seven domains, i.e. a serine protease domain at the C terminus, a Pan Apple (PAp) domain at the N terminus, and five Kringle domains (Kringle1 to Kringle5). Referring to sequences in Swiss Prot, the signal peptide includes residues Met1-Gly19, PAp includes residues Glu20-Val98, Kringle1 includes residues Cys103-Cys181, Kringle2 includes residues Glu184-Cys262, Kringle3 includes residues Cys275-Cys352, Kringle4 includes residues Cys377-Cys454, and Kringle5 includes residues Cys481-Cys560. According to data of NCBI, the serine protease domain includes residues Val581-Arg804.

Glu-plasminogen is human natural full-length plasminogen and is composed of 791 amino acids (without a signal peptide of 19 amino acids), a cDNA sequence for encoding the sequence is shown as sequence 1, and an amino acid sequence of Glu-plasminogen is shown as sequence 2. In vivo, there is Lys-plasminogen formed by hydrolyzing Glu-plasminogen at amino acids at the 76th site and the 77th site, which is shown as sequence 6, and a cDNA sequence for encoding the amino acid sequence is shown as sequence 5. Delta-plasminogen (δ-plasminogen) is full-length plasminogen lacking a fragment from Kringle2 to Kringle5 and containing only Kringle1 and a serine protease domain (also referred to as a protease domain (PD)), an amino acid sequence (sequence 8) of delta-plasminogen has been reported in a document, and a cDNA sequence for encoding the amino acid sequence is shown as sequence 7. Mini-plasminogen is composed of Kringle5 and a serine protease domain, it has been reported in a document that an amino acid sequence of mini-plasminogen includes residues Val443-Asn791 (taking a Glu residue in a sequence of Glu-plasminogen without a signal peptide as the starting amino acid), and is shown as sequence 10, and a cDNA sequence for encoding the amino acid sequence is shown as sequence 9. Micro-plasminogen contains only a serine protease domain, it has been reported in a document that an amino acid sequence of micro-plasminogen includes residues Ala543-Asn791 (taking a Glu residue in a sequence of Glu-plasminogen without a signal peptide as the starting amino acid), and it has also been reported in the patent document CN102154253A that the sequence of plasminogen includes residues Lys531-Asn791 (taking a Glu residue in a sequence of Glu-plasminogen without a signal peptide as the starting amino acid). In this patent application, the amino acid sequence of micro-plasminogen is referred to the patent document CN102154253A, and is shown as sequence 12, and a cDNA sequence for encoding the amino acid sequence is shown as sequence 11.

The structure of full-length plasminogen is also described in the paper of Aisina, et al. (Aisina R B, Mukhametova L 1. Structure and function of plasminogen/plasmin system [J]. Russian Journal of Bioorganic Chemistry, 2014, 40 (6): 590-605). In this paper, Aisina, et al. describe that plasminogen includes Kringle 1, 2, 3, 4, and 5 domains and a serine protease domain (also referred to as a protease domain (PD)). Kringles are responsible for binding plasminogen to ligands having low molecular wights and high molecular weights (i.e. the lysine binding activity), so that plasminogen is transformed into a more open conformation, which can be activated more easily. The protease domain (PD) includes residues Val562-Asn791, and tPA and uPA specifically cleave an activation bond at sites Arg561-Val562 in plasminogen to transform plasminogen into plasmin. Therefore, the protease domain (PD) is a region giving the proteolytic activity of plasminogen.

Herein, the terms "plasmin", "fibrinolysin", and "fibrinolytic enzyme" are interchangeable and have the same meaning. The terms "plasminogen", "profibrinolysin", and "fibrinolytic zymogen" are interchangeable and have the same meaning.

In the present invention, the "plasminogen deficiency" means that the plasminogen content or activity in a subject is less than that in a normal person, and is low enough to affect normal physiological functions of the subject. The "plasminogen deficiency" means that the plasminogen content or activity in a subject is less than that in a normal person, the activity or expression of plasminogen is extremely low, and the normal physiological functions can only be maintained by providing exogenous plasminogen.

Those in the art may understand that all technical solutions of plasminogen of the present invention are applicable to plasmin, and thus the technical solutions described in the present invention cover plasminogen and plasmin. During circulation, plasminogen is in a closed inactive conformation, and is transformed into activate plasmin in an open conformation under the mediation of a plasminogen activator (PA) when binding to a thrombus or cell surface. Active plasmin can further hydrolyze a fibrin clot to fibrin degradation products and D-dimer, so as to dissolve a thrombus. The PAp domain of plasminogen contains an important determinant for maintaining plasminogen in a closed inactive conformation, and the KR domain of plasminogen can bind to a lysine residue present in a receptor and a substrate. A variety of known enzymes that can be used as plasminogen activators include: a tissue-type plasminogen activator (tPA), a urokinase-type plasminogen activator (uPA), a kallikrein, a blood coagulation factor XII (Hageman factor), etc.

An "active plasminogen fragment" refers to a fragment having the activity of binding to lysine in a target sequence of a substrate (the lysine binding activity), or the activity of exerting proteolytic function (the proteolytic activity), or the proteolytic activity and the lysine binding activity. The technical solutions related to plasminogen of the present invention cover a technical solution of replacing plasminogen with an active plasminogen fragment. In some embodiments, the active plasminogen fragment of the present invention contains the serine protease domain of plasminogen or is composed of the serine protease domain of plasminogen. In some embodiments, the active plasminogen fragment of the present invention contains sequence 14, or contains an amino acid sequence having at least 80%, 90%, 95%, 96%, 97%, 98% or 99% identity with sequence 14, or is composed of sequence 14, or is composed of the amino acid sequence having at least 80%, 90%, 95%, 96%, 97%, 98% or 99% identity with sequence 14. In some embodiments, the active plasminogen fragment of the present invention contains one or more regions selected from Kringle 1, Kringle 2, Kringle 3, Kringle 4, and Kringle 5 or conservatively substituted variants thereof, or is composed of one or more regions selected from Kringle 1, Kringle 2, Kringle 3, Kringle 4, and Kringle 5 or conservatively substituted variants thereof. In some embodiments, the plasminogen of the present invention includes a protein containing the above active plasminogen fragment.

At present, assays of plasminogen in the blood and its activity include: an tissue-type plasminogen activator activity assay (t-PAA), a plasma tissue-type plasminogen activator antigen assay (t-PAAg), a plasma tissue-type plasminogen activity assay (plgA), a plasma tissue plasminogen antigen assay (plgAg), a plasma tissue-type plasminogen activator inhibitor activity assay, a plasma tissue-type plasminogen activator inhibitor antigen assay, and a plasma plasmin-antiplasmin complex assay (PAP). The most commonly used test method is a chromogenic substrate method: streptokinase (SK) and a chromogenic substrate are added to plasma to be tested, PLG in the plasma to be tested is transformed into PLM under the action of SK, PLM acts on the chromogenic substrate, absorbance is measured by using a spectrophotometer, and increase in absorbance is proportional to the activity of plasminogen. In addition, immunohistochemistry, gel electrophoresis, immunoturbidimetry, radial immunodiffusion, etc. can also be adopted to test the activity of plasminogen in the blood.

An "ortholog" refers to a homolog of different species, includes a protein homolog and a DNA homolog, and is also referred to as a vertical homolog. It specifically refers to a protein or a gene in different species that has evolved from the same ancestral gene. The plasminogen of the present invention includes human natural plasminogen, and also includes plasminogen orthologs derived from different species and having the activity of plasminogen.

A "conservatively substituted variant" refers to that a given amino acid residue is changed, but the whole conformation and function of a protein or enzyme are not changed. For example, an amino acid in an amino acid sequence of a parent protein is substituted with an amino acid with similar properties (e.g. acidity, alkalinity, and hydrophobicity). The amino acid with similar properties is well known. For example, arginine, histidine, and lysine are hydrophilic alkaline amino acids and can be substituted with each other. Similarly, isoleucine is a hydrophobic amino acid and can be substituted with leucine, methionine or valine. Therefore, the similarity of amino acid sequences of two protein having similar functions may be different. For example, 70% to 99% similarity (identity) based on the MEGALIGN algorithm. The "conservatively substituted variant" also includes a polypeptide or an enzyme that has more than 60% amino acid sequence identity determined based on BLAST or FASTA algorithm, preferably, more than 75% identity, more preferably, more than 85% identity, and the most preferably, more than 90% identity. The polypeptide or the enzyme has the same or basically similar properties or function compared to a natural or parent protein or enzyme.

"Isolated" plasminogen refers to a plasminogen protein isolated and/or recovered from a natural environment of plasminogen. In some embodiments, the plasminogen is purified (1) to the purity (by weight) of more than 90%, more than 95% or more than 98%, such as more than 99% determined by the Lowry method, (2) to an extent sufficient to obtain at least 15 residues at the N terminus or in an internal amino acid sequence by using a rotary cup sequencer, or (3) to homogeneity that is determined by sodium dodecyl sulfate-polyacrylamide gel electrophoresis (SDS-PAGE) using Coomassie blue or silver staining under reducing or non-reducing conditions. The isolated plasminogen also includes plasminogen prepared from a recombinant cell by a bioengineering technology and isolated by at least one purification step.

Herein, the terms "polypeptide", "peptide", and "protein" are interchangeable, refer to an aggregation form of amino acids of any length, and may include genetically encoded and nongenetically encoded amino acids, chemically or biochemically modified or derived amino acids, and a polypeptide having a modified peptide backbone. The terms include fusion proteins, which include, but are not limited to, a fusion protein having an heterogenous amino acid sequence, a fusion having heterogenous and homologous leader sequences (having or without an N-terminal methionine residue), etc.

"Amino acid sequence identity percentage (%)" relative to a reference polypeptide sequence is defined as, after gaps have been introduced as necessary to achieve the maximum percentage sequence identity, and no conservative substitutions are considered as a part of the sequence identity, the percentage of amino acid residues, which are identical to amino acid residues in the reference polypeptide sequence, in a candidate sequence. Comparison for determining percentage amino acid sequence identity can be achieved in a variety of ways within the technical scope of the art. For example, software available to the public, such as BLAST, BLAST-2, ALIGN, and Megalign (DNASTAR), is adopted. Those skilled in the art can determine appropriate parameters used for comparing sequences, such as any algorithm needed to achieve the maximum comparison over the full lengths of sequences to be compared. However, for the purpose of the present invention, an amino acid sequence identity percentage value is generated by using the sequence comparison computer program ALIGN-2.

In a case that ALIGN-2 is adopted to compare amino acid sequences, % amino acid sequence identity of a given amino acid sequence A relative to a given amino acid sequence B (or may be expressed as that the given amino acid sequence A has or contains certain % amino acid sequence identity relative to, with, or to the given amino acid sequence B) is calculated as follows:

$$\text{Score } X/Y \text{ multiply } 100$$

where, X is the number of amino acid residues, identically matched with amino acid residues in B, in A that is determined by the sequence comparison program ALIGN-2, and Y is the total number of amino acid residues in B. It is to be understood that in a case that the length of the amino acid sequence A differs from that of the amino acid sequence B, % amino acid sequence identity of A relative to B is not equal to % amino acid sequence identity of B relative to A. Unless otherwise specifically described, all % amino acid sequence identity values used herein are obtained by using the ALIGN-2 computer program as described in the preceding paragraph.

As used herein, the term "treatment" refers to obtaining a desired pharmacological and/or physiological effect. The effect may be complete or partial prevention of occurrence and onset of a disease or its symptoms, partial or complete alleviation of a disease and/or its symptoms, and/or partial or complete cure of a disease and/or its symptoms, which includes: (a) prevention of occurrence or onset of a disease in a subject who may have a predisposition to the disease but has not been diagnosed with the disease; (b) inhibition of a disease, i.e. retardation of the formation of the disease; and (c) alleviation of a disease and/or its symptoms, i.e. subsidence or disappearance of the disease and/or its symptoms.

Herein, the terms "individual", "subject", and "patient" are interchangeable, and refer to a mammal, which includes, but is not limited to, murine (rats and mice), non-human primates, humans, dogs, cats, ungulates (e.g. horses, cattle, sheep, pigs, and goats), etc.

A "therapeutically effective amount" or "effective dose" refers to an amount of plasminogen activation pathway components or its related compound (e.g. plasminogen) that is sufficient to prevent and/or treat a disease when administered to a mammal or other subjects to treat the disease. The "therapeutically effective amount" is changed with plasminogen activation pathway components or its related compound (e.g. plasminogen) used, a disease of a subject to be treated and/or the severity of symptoms, age, and weight, etc.

Preparation of the plasminogen of the present invention

Plasminogen can be isolated from nature and purified for further therapeutic use, or can be synthesized by a standard chemical peptide synthesis technology. In a case that a polypeptide is synthesized chemically, plasminogen can be synthesized from a liquid phase or a solid phase. Solid-phase polypeptide synthesis (SPPS) (in which a C-terminal amino acid of a sequence is attached to an insoluble support, followed by sequential addition of the remaining amino acids in the sequence) is a method suitable for chemical synthesis of plasminogen. Various SPPS methods, such as Fmoc and Boc, can be used to synthesize plasminogen. The solid-phase synthesis technique is described in Barany, et al. Solid-Phase Peptide Synthesis; The Peptides: Analysis, Synthesis, Biology. Vol. 2: Special Methods in Peptide Synthesis, Part A, 3-284; Merrifield. Solid Phase Peptide Synthesis. I. The Synthesis of a Tetrapeptide. J. Am. Chem. Soc., 85: 2149-2156 (1963); Stewart, et al. Solid Phase Peptide Synthesis, 2nd ed. Pierce Chem. Co., Rockford, Ill. (1984); Ganesan A. 2006 Mini Rev. Med Chem. 6: 3-10; and Camarero JA, et al. 2005 Protein Pept Lett. 12: 723-8. In short, small insoluble porous beads are treated with a functional unit on which a peptide chain is built. After recirculation of coupling/deprotection, the attached solid-phase free N-terminal amine is coupled to a single N-protected amino acid unit. This unit is then deprotected to reveal a new N-terminal amine that can be attached to another amino acid. The peptide remains immobilized on the solid phase and then is cleaved.

Plasminogen of the present invention can be produced by a standard recombination method. For example, a nucleic acid for encoding plasminogen is inserted into an expression vector so as to be operably connected to a regulatory sequence in the expression vector. The expression regulatory sequence includes, but is not limited to, a promoter (e.g. a naturally related or heterogenous promoter), a signal sequence, an enhancer element, and a transcription termination sequence. The expression regulatory sequence may be a eukaryotic promoter in the vector, and the vector can transform or transfect eukaryotic host cells (e.g. COS or CHO cells). Once the vector is incorporated into a suitable host, the vector maintains the host under conditions suitable for high expression of a nucleotide sequence and collection and purification of plasminogen.

The suitable expression vector usually replicates in the host organism as an episome or an integrated part of the host chromosomal DNA. Normally, the expression vector contains a selectable marker (e.g. ampicillin resistance, hygromycin b resistance, tetracycline resistance, kanamycin resistance, and neomycin resistance) to facilitate detection of cells transformed with an exogenous desired DNA sequence.

Exemplary prokaryotic host cells that can be used to clone a polynucleotide for encoding a subject antibody include Escherichia coli. Other suitable microbial hosts include: Bacillus such as Bacillus subtilis, and other Enterobacteriaceae such as Salmonella, Serratia, and various Pseudomonas species. Expression vectors can also be generated in these prokaryotic hosts, and usually contain expression control sequences (origin of replication) compatible with the host cells. In addition, there are many known promoters, such as a lactose promoter system, a tryptophan (trp) promoter system, a beta-lactamase promoter system, and a promoter system from bacteriophage $\lambda$. The promoter usually controls expression optionally in a sequence of an operator gene, and has a ribosome binding site sequence for initiating and completing transcription and translation.

Other microorganisms, such as yeast, can also be used for expression. Exemplary suitable yeast host cells include yeast (e.g. Saccharomyces cerevisiae (S. cerevisiae)) and Pichia, and the suitable vector has an expression control sequence (e.g. a promoter), origin of replication, a terminator sequence, etc. according to the requirements. Typical promoters contain 3-phosphoglycerate kinase and other glycogenolysis enzymes. Inducible yeast promoters specially include promotes from alcohol dehydrogenase, hetero-cytochrome C, and enzymes responsible for using maltose and galactose.

In addition to microorganisms, mammalian cells (e.g. mammalian cells cultured in a cell culture medium in vitro) can also be used for expressing and generating the anti-Tau antibody (e.g. a polynucleotide for encoding a subject anti-Tau antibody) of the present invention. Referring to Winnacker, From Genes to Clones, VCH Publishers, N.Y., N.Y. (1987). Suitable mammal host cells include a CHO cell line, various Cos cell line, HeLa cells, a myeloma cell line, and transformed B cells or hybridoma. An expression vector used for these cells may include an expression control sequence such as origin of replication, a promoter, and an enhancer (Queen, et al. Immunol. Rev. 89: 49 (1986)), and a necessary processing information site such as a ribosome binding site, an RNA splicing site, a polyadenylation site, and a transcription terminator sequence. Exemplary suitable expression control sequences include derived promoters such as a white immunoglobulin gene, SV40, an adenovirus, a bovine papillomavirus, and a cytomegalovirus Referring to Co, et al. J. Immunol. 148: 1149 (1992).

Once the plasminogen of the present invention is synthesized (by the chemical or recombination method), the plasminogen of the present invention is purified in accordance with the standard procedure in the art, which includes ammonium sulfate precipitation, affinity column chromatography, high performance liquid chromatography (HPLC), gel electrophoresis, etc. The plasminogen is substantially pure, for example, at least about 80% to 85% pure, at least 85% to 90% pure, at least about 90% to 95% pure, 98% to 99% pure or purer. For example, the plasminogen does not contain contaminants such as cellular debris and macromolecules other than the target product.

Drug preparation

A therapeutic preparation is a lyophilized preparation or an aqueous solution formed by mixing plasminogen activation pathway components or its related compound (e.g. plasminogen) with required purity with an optional pharmaceutical carrier, an excipient or a stabilizer (Remington's Pharmaceutical Sciences, 16th Edition, Osol, A. ed. (1980)). The acceptable carrier, excipient or stabilizer at a used dose and concentration is nontoxic to subjects, and include a buffer such as phosphates, citrates, and other organic acids; an antioxidant such as ascorbic acid and methionine; a preservative (e.g. octadecyl dimethyl benzyl ammonium chloride, hexanediamine chloride, benzalkonium chloride, benzethonium chloride, phenol, butanol, benzyl alcohol, alkyl parabens such as methyl and ethyl parabens, catechol, resorcinol, cyclohexanol, 3-pentanol, and m-cresol); a polypeptide with a low molecular weight (less than about 10 residues); a protein such as serum albumin, gelatin, and immunoglobulin; a hydrophilic polymer such as polyvinylpyrrolidone; an amino acid such as glycine, glutamine, asparagine, histidine, arginine, and lysine; monosaccharide, disaccharide, and other carbohydrates such as glucose, mannose, and dextrin; a chelant such as EDTA; saccharides such as sucrose, mannitol, fucose, and sorbitol; salt-forming counterions such as sodium; a metal complex (e.g. a zinc-protein complex); and/or a non-ionic surfactant such as TWEENTM, PLURONICSTM, and polyethylene glycol (PEG). A preferred lyophilized anti-VEGF antibody preparation is described in WO 97/04801, which is incorporated herein by reference.

The preparation of the present invention may also contain more than one active compound needed for treating a specific symptom, and preferably, the active compounds are complementary and do not have side effects on each other.

The plasminogen of the present invention can be encapsulated in a microcapsule prepared by techniques such as coacervation or interfacial polymerization, for example, can be placed in a colloidal drug delivery system (e.g. a liposome, an albumin microsphere, a microemulsion, nanoparticles, and a nanocapsule) or placed in hydroxymethylcellulose in a macroemulsion or a gelmicrocapsule and a poly-(methyl methacrylate) microcapsule. These techniques are disclosed in Remington's Pharmaceutical Sciences 16th edition, Osol, A. Ed. (1980).

Plasminogen activation pathway components or their related compounds (e.g. plasminogen) used for in vivo administration need to be sterile. It can be easily achieved by filtration with a sterile filter before or after lyophilization and re-preparation.

Plasminogen activation pathway components or their related compounds (e.g. plasminogen) of the present invention can be used for preparation of a sustained-release preparation. Exemplary suitable sustained-release preparations include semipermeable matrices of solid hydrophobic polymers having a shape and containing glycoproteins, such as membranes or microcapsules. Exemplary sustained-release matrices include polyesters, hydrogels (e.g. poly(2-hydroxyethylmethacrylate) (Langer, et al. J. Biomed. Mater. Res., 15: 167-277 (1981); Langer, Chem. Tech., 12: 98-105 (1982)) or polyvinyl alcohol, polylactide (US patent 3773919, EP 58,481), L-glutamic acid and a copolymer of $\gamma$ ethyl-L-glutamic acid (Sidman, et al. Biopolymers 22: 547 (1983)), nondegradable ethylene-vinyl acetate (Langer, et al. same as above) or degradable lactic acid-glycolic acid copolymer such as Lupron Depot™ (an injectable microsphere composed of a lactic acid-glycolic acid copolymer and leuprolide acetate), and poly-D-(-)-3-hydroxybutyric acid. The compounds, such as ethylene-vinyl acetate and lactic acid-glycolic acid, can sustainably release molecules for more than 100 days, and some hydrogels release proteins for a short time period. Rational strategies for stabilizing proteins can be designed based on the relevant mechanisms. For example, in a case where the mechanism of coacervation is formation of intermolecular S-S bonds through the exchange of thiodisulfide bonds, proteins can be stabilized by modifying sulfhydryl residues, lyophilizing from an acid solution, controlling humidity, using an appropriate additive, and developing a specific polymer matrix composition.

Administration and dosage

The pharmaceutical composition of the present invention can be administered by different methods, such as nasal inhalation, aerosol inhalation, nasal drops or eye drops, an intravenous method, an intraperitoneal method, a subcutaneous method, an intracranial method, an intrathecal method, an intraarterial method (e.g. via the carotid artery), an intramuscular method, and a rectal administration.

Preparations for parenteral administration include sterile aqueous or non-aqueous solutions, suspensions, and emulsions. Non-aqueous solvents include propylene glycol, polyethylene glycol, vegetable oils such as olive oil, and injectable organic esters such as ethyl oleate. Aqueous vectors include water, alcoholic/aqueous solutions, emulsions, and suspensions such as saline and a buffer medium. Parenteral intermedia include a sodium chloride solution, Ringer's dextrose, dextrose, sodium chloride, and fixed oils. Intravenous intermedia include fluids and nutritional supplements, electrolyte supplements, etc. A preservative and other additives such as an antimicrobial, an antioxidant, a chelator, and inert gas, may be present.

Medical staffs will determine a dosage regimen based on various clinical factors. As well known in the medical field, a dosage regimen for any patient is determined according to a variety of factors, including the body size of a patient, the body surface area, age, a specific compound to be administered, sex, the frequency and path of administration, general health conditions, and other drugs to be administered together. A daily dosage range of the pharmaceutical composition containing plasminogen of the present invention may be, for example, about 0.0001-2000 mg/kg, or about 0.001-500 mg/kg (e.g. 0.02 mg/kg, 0.25 mg/kg, 0.5 mg/kg, 0.75 mg/kg, 10 mg/kg, and 50 mg/kg) of body weight of a patient. For example, a dose may be 1 mg/kg of body weight or 50 mg/kg of body weight, or within a range of 1-50 mg/kg of body weight, or at least 1 mg/kg of body weight. Doses greater or less than these exemplary ranges are also covered, especially in view of the above factors. Intermediate doses within the above ranges also fall within the scope of the present invention. Subjects may be administered with the pharmaceutical composition at such doses daily, every other day, weekly, or according to any other regimen determined by empirical analysis. Exemplary dosage regimens include that the pharmaceutical composition is administered at 0.01-100 mg/kg for consecutive days. It is necessary to assess a therapeutic effect and the safety during administration with the drug of the present invention.

Product or kit

An embodiment of the present invention relates to a product or kit, which includes plasminogen activation pathway components or its related compound (e.g. plasminogen). Preferably, the kit includes a container with a label or package insert. Suitable containers include bottles, vials, syringes, etc. The container can be made from a variety of materials such as glass and plastic. The container contains a composition that can be used to treat the disease or symptoms of the present invention, and has a sterile inlet (e.g. the container may be an intravenous solution pack or vial with a plug that can be penetrated by a hypodermic needle). At least one active ingredient in the composition is plasminogen activation pathway components or its related compound (e.g. plasminogen). The label attached to the container is used to describe that the composition is used to treat the symptoms of the present invention. The product may also include a second container containing a medicinal buffer such as phosphate-buffered saline, a Ringer's solution, and a dextrose solution. The product may also include other substances required from a commercial and user standpoint, which include other buffers, a diluent, a filter, a needle, and a syringe. In addition, the product includes a package insert with instructions for use, which are used to, for example, indicate a user of the composition to administrate plasminogen activation pathway components or its related compound (e.g. plasminogen) and other drugs for treating concomitant diseases to a patient.

Examples

Human plasminogen used in all the following examples is derived from plasma of human donors based on a method described in the following literature (KennethC Robbins, Louis Summaria, David Elwyn et al. Further Studies on the Purification and Characterization of Human Plasminogen and Plasmin. Journal of Biological Chemistry, 1965, 240 (1) :541-550; Summaria L, Spitz F, Arzadon L et al. Isolation and characterization of the affinity chromatography forms of human Glu- and Lysplasminogens and plasmins. J Biol Chem. 1976 Jun 25; 251(12):3693-9; HAGAN JJ, ABLONDI FB, DE RENZO EC. Purification and biochemical properties of human plasminogen. J Biol Chem. 1960 Apr; 235:1005-10), subjected to process optimization and purified from the plasma of the human donors, wherein a content of a plasminogen monomer is larger than 98%.

**Example 1 Plasminogen promotes degradation of recombinant human α-synuclein in cerebral homogenates of mouse models of Parkinson's disease**

Eight C57BL/6J male mice aged 11-12 weeks with a body wight of 18-25 g were taken and weighed one day before modeling. The mice were randomly divided into two groups according to body weights with 4 in a blank control group and 4 in a model group. Modeling was timed at 9: 00 am every time; 200 μL of normal saline was intraperitoneally injected into the mice in the control group; 5 mg/kg mL MPTP (1-methyl-4-phenyl-1, 2, 3, 6-tetrahydropyridine, 1-methyl-4-phenyl-1, 2, 3, 6-tetrahydropyridine) solution was intraperitoneally injected into the mice in the model group by 35 mg/kg per mouse for consecutive 5 days; and a Parkinson's disease model was established[1]. Preparation of the MPTP solution: 45 mg of MPTP (sigma, M0896) was dissolved into 9 mL normal saline solution to prepare the final concentration as 5 mg/mL. On the 6th day after the completion of modeling, all mice were subjected to open field test to identify the success of modeling. After all the mice were sacrificed, whole brains were taken, weighed and was added to 1×PBS (Thermo Fisher, pH 7.4; 10010-031) according to 150 mg tissue/mL PBS for homogenization at 4°C (1 min/time, 3-4 times); then centrifugation was conducted at 4 °C (12000 rpm, 20 min); and supernatants (brain tissue homogenate) were taken and placed in EP tubes.

Eppendorf (EP) tubes were divided into ① a blank control group, ② a solvent control group, and ③ a plasminogen group, and 5 parallels were set for each group. 21.5 μL of normal saline, 4.6 μL of plasminogen solution (2 mg/mL) and 23.9 μL of cerebral homogenate of mice were added to the blank control group; 21.5 μL of α-synuclein solution (1.0 mg/mL, customerized expressed human α-synuclein, ChinaPeptides, uniProtKB-P37840), 4.6 μL of solvent solution (10 mM sodium citrate, 2% arginine hydrochloride, 3% mannitol, pH 7.4) and 23.9 μL of cerebral homogenate of the mice were added to the solvent control group; and 21.5 mL of α-synuclein (1.0 mg/mL), 4.6 μL of plasminogen solution (2 mg/mL) and 23.9 μL of cerebral homogenate of the mice were added to the plasminogen group. Then, the materials in each tube were incubated at 37°C for 6 h, and 50 μL of 0.1% trifluoroacetic acid solution was placed in each tube to terminate the reaction.

A 12% gel was prepared according to instructions of a Tris-Tricine-SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 1.5 h, and then at 100 V to the bottom of the gel. After electrophoresis was completed, the gel was peeled off, stained with a 1‰ Coomassie brilliant blue staining solution (1 g of coomassie brilliant blue R250 was dissolved in 1000 mL of mixture of ethanol, acetic acid, and purified water in a volume ratio of 5: 2: 13) for 30 min, and destained with a destaining solution (a mixture of purified water, acetic acid, and anhydrous ethanol in a volume ratio of 17: 2: 1) to clear. The gel was photographed by using a biomolecular imager and subjected to quantitative scanning analysis.

At present, the Parkinson's disease is thought to be caused by loss of dopaminergic neurons in substantia nigra of midbrain and appearance of Lewy bodies, α-synuclein is a neuronal protein composed of 140 amino acid residues, may cause a neuronal damage and participates in the neurodegenerative process of a central nervous system. Researches have shown that the Lewy bodies of nerve cells and the α-synuclein aggregating in nerve synapse are markers of brain diseases in the Parkinson's disease[2].

The results show that in the cerebral homogenates of the mouse models of Parkinson's disease, the amount of α-synuclein in the plasminogen group is significantly lower than that in the solvent control group, and the difference is very significant (*** indicates P<0.001); and the amounts of aggregates a and b in the plasminogen group are significantly lower than those in the solvent control group, and the differences are very significant (** indicates P<0.01, *** indicates P<0.001). In the cerebral homogenates of the normal mice, the amount of α-synuclein in the plasminogen group is significantly lower than that in the solvent control group, and the difference very significant (*** indicates P<0.001); and the amounts of aggregates a and b are significantly lower than those in the solvent control group, and the differences are significant (* indicates P<0.05, ** indicates P<0.01) (FIG. 1). It is shown that the plasminogen may effectively degrade human α-synuclein and the aggregates thereof in the cerebral homogenates of the mouse models of Parkinson's disease and the normal mice.

**Example 2 Plasminogen promotes degradation of α-synuclein in cerebral homogenates of mouse models of Parkinson's disease**

Eight C57BL/6J male mice aged 11-12 weeks with a body wight of 18-25 g were taken and weighed one day before modeling. The mice were randomly divided into two groups according to body weights with 4 in a blank control group and 4 in a model group. As described in example 1, a Parkinson's disease model[5] was established, and cerebral homogenates were prepared and placed in EP tubes.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (10 mM sodium citrate, 2% arginine hydrochloride, 3% mannitol, pH 7.4) and 23.9 μL of cerebral homogenate of the mice were added to the blank group; 21.5 μL of normal saline, 4.6 μL of plasminogen solution (2 mg/mL) and 23.9 μL of cerebral homogenate of the mice were added to the blank control group; 21.5 μL of α-synuclein (1.0 mg/mL, customized expressed human α-synuclein, ChinaPeptides, UniProtKB-P37840), 4.6 μL of solvent solution and 23.9 μL of cerebral homogenate of the mice were added to the solvent control group; and 21.5 mL of α-synuclein (1.0 mg/mL), 4.6 μL of plasminogen solution (2 mg/mL) and 23.9 μL of cerebral homogenate of the mice were added to the plasminogen group. Then, the materials in each tube were incubated at 37 °C for 6 h, and 50 μL of 0.1% trifluoroacetic acid solution was placed in each tube to terminate the reaction.

A 12% gel was prepared according to instructions of a Tris-Tricine-SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 1.5 h, and then at 100 V to the bottom of the gel. After electrophoresis was completed, the gel was peeled off and transferred to a PVDF membrane (GE, A29433753), and electrophoresis was performed at 15 V for 2 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human α-synuclein antibody (Proteintech, 10842-1-AP) was added, the PVDF membrane was incubated at the room temperature for 3 h, and washed four times with TBST, a goat anti-rabbit IgG (HRP) antibody (Abeam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that in the cerebral homogenates of the mouse models of Parkinson's disease, the amount of α-synuclein in the plasminogen group is significantly lower than that in the solvent control group with a very significant difference (\*\* indicates P<0.01), and the amount of an aggregate of α-synuclein in the plasminogen group is significantly lower than that in the solvent control group with a very significant difference (\*\*\* indicates P<0.001); and in the cerebral homogenates of the normal mice, the amount of α-synuclein in the plasminogen group is significantly lower than that in the solvent control group with a very significant difference (\*\* indicates P<0.01), and the amount of the aggregate of the α-synuclein is significantly lower than that in the solvent control group with a significant difference (\*\*\* indicates P<0.001) (FIG. 2). It is shown that in the cerebral homogenates of the mouse models of Parkinson's disease and the normal mice, the plasminogen may effectively degrade human α-synuclein and the aggregate thereof.

**Example 3 Plasminogen may decrease expression level of α-synuclein in substantia nigra of mouse models of Parkinson's disease**

Twenty-eight C57BL/6J male mice aged 10-12 weeks were taken and weighed one day before modeling; and the mice were randomly divided into two groups according to body weight with 8 in a blank control group and 20 in a model group. The Parkinson's disease model[1] was established as described in example 1. On the 6th day after the completion of modeling, the mice in the model group were randomly divided into two groups according to body weight: a solvent group and an administration group, with 10 mice in each group; the mice in the administration group were injected with 1 mg/100 μL plasminogen solution per mouse via a tail vein, while the mice in the solvent group were injected with 100 μL of solvent solution per mouse for consecutive 14 days; and the mice were sacrificed on Day 15 after administration, and substantia nigra of the mice were sampled and fixed in 4% paraformaldehyde for 24-48 hours. The fixed brain tissue was dehydrated with graded ethanol, cleared with xylene, and embedded in paraffin. The substantia nigra in a slice with a thickness of 3 μm was positioned, and the slice was subjected to deparaffinage and rehydration, and then washed once with water. The slice was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse α-synuclein antibody (Proteintech, 10842-1-AP) was added dropwise, and the slice was incubated at 4 °C overnight, and washed twice with 0.01 M PBS for 5 min each time. A goat anti-rabbit IgG (HRP) antibody (Abcam) secondary antibody was added, and the slice was incubated at the room temperature for 1 h, and washed twice with 0.01 M PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 400× optical microscope.

The results show that there is only a small amount of α-synuclein in the substantia nigra of the mice in the blank control group (FIG. 3A); the amount of α-synuclein in the substantia nigra of the mice in the solvent group (FIG. 3B) is significantly higher than that in the blank control group (\* indicates P<0.05); and the amount of α-synuclein in the substantia nigra of the mice in the plasminogen administration group (FIG. 3C) is significantly lower than that in the solvent group and approaches that in the blank control group, with a statistically significant difference (\* indicates P<0.05) (FIG. 3D). It is shown that the plasminogen may reduce an expression level of α-synuclein in the substantia nigra of the mouse models of Parkinson's disease and improve degeneration of neurons.

**Example 4 Plasminogen can promote the degradation of human amyloid (Aβ40) in a PBS buffer system**

Eppendorf (EP) tubes were divided into ① a blank control group, ② a solvent control group, ③ a plasminogen group, and ④ a plasminogen+tPA group, 4 tubes in each group. 43.3 μL of normal saline, 16 μL of plasminogen solution (0.575 mg/mL), 10 μL of ultra-pure water, and 30.7 μL of PBS (10 mM, pH=7.4, Thermo Fisher, 10010-031) were placed in each tube of the blank control group. 43.3 μL of Aβ40 (1.0 mg/mL, ChinaPeptides, 04010011521), 16 μL of solvent solution (10 mM sodium citrate, 2% arginine hydrochloride, and 3% mannitol, pH=7.4), 10 μL of ultra-pure water, and 30.7 μL of PBS were placed in each tube of the solvent control group. 43.3 μL of Aβ40 (1.0 mg/mL), 16 μL of plasminogen solution (0.575 mg/mL), 10 μL of ultra-pure water and 30.7 μL of PBS were placed in each tube of the plasminogen group. 43.3 μL of Aβ40 (1.0 mg/mL), 8 μL of plasminogen solution (1.15 mg/mL), 8 μL of tPA solution (1.0 mg/mL), 10 μL of lysine solution (0.1 mM) and 30.7 μL of PBS were placed in each tube of the plasminogen+tPA group. Then, the materials in each tube were incubated at 37°C for 3 h, and 100 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 1 min, and 20 μL of sample was loaded. Electrophoresis was performed at 30 V for 1 h, and then at 100 V to the bottom of the gel. After electrophoresis was completed, the gel was peeled off and stained with a 1‰ Coomassie brilliant blue staining solution (1 g of Coomassie brilliant blue R250 was dissolved in 1000 mL of mixture of ethanol, glacial acetic acid, and purified water in a volume ratio of 5: 2: 13) for 30 min, and destained with a destaining solution (a mixture of purified water, glacial acetic acid, and anhydrous ethanol in a volume ratio of 17: 2: 1) to clear. The gel was photographed and quantitatively scanned by using a biomolecular imager.

The accumulation of amyloid beta-protein (Aβ) is a key factor in the formation of Alzheimer's disease. Aβ40 and Aβ42 containing 40 and 42 residues constitute the main morphology of senile plaques, i.e. their deposition in the hippocampus and striatum of the brain is the main pathogenic factor causing AD[3]. The Aβ40 content and the Aβ42 content in the cerebrospinal fluid have gradually become physiological indicators for clinical diagnosis of Alzheimer's disease.

The results show that the amount of Aβ40 in the solvent control group is defined as 100% and does not have any change; Aβ40 in the plasminogen group is partially degraded in a case that plasminogen is added alone; and Aβ40 in the plasminogen+tPA group is obviously degraded in vitro in a case that plasminogen and tPA are added, and the difference between the plasminogen+tPA group and the solvent control group is significant (** indicates P<0.01) (FIG. 4). It is indicated that plasminogen can promote the degradation of Aβ40 in a PBS buffer system.

**Example 5 Plasminogen can promote the degradation of human amyloid (Aβ40) in the cerebrospinal fluids of rabbits**

Eppendorf (EP) tubes were divided into ① a blank control group, ② a solvent control group, ③ a plasminogen group, and ④ a plasminogen+tPA group. In the blank control group, 43.3 μL of normal saline, 16 μL of plasminogen solution (0.575 mg/mL) and 40.7 μL of cerebrospinal fluid of rabbits (commercially available) were added; in the solvent control group, 43.3 μL of Aβ40 (1.0mg/mL, ChinaPeptides, 04010011521), 16 μL of solvent solution (10 mM sodium citrate, 2% arginine hydrochloride, 3% mannitol, pH 7.4) and 40.7 μL of cerebrospinal fluid of rabbits were added; in the plasminogen group, 43.3 μL of Aβ40 (1.0 mg/mL), 16 μL of plasminogen solution (0.575 mg/mL) and 40.7 μL of cerebrospinal fluid of rabbits were added; in the plasminogen+tPA group, 43.3 μL of Aβ40 (1.0 mg/mL), 8 μL of plasminogen solution (1.15 mg/mL), 8 μL of tPA solution (1.0 mg/mL) and 40.7 μL of PBS buffer were added. Then, the materials in each tube were incubated at 37°C for 3 h, and 100 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). Each group of samples was electrophoresed as described in example 1, stained with Coomassie brilliant blue, then destained and scanned quantitatively.

The results show that the amount of Aβ40 in the solvent control group is defined as 100% and does not change; and Aβ40 in the plasminogen group is partially degraded in a case where plasminogen is added alone and degraded to 74.81% (FIG. 5). It is indicated that plasminogen can promote the degradation of human amyloid Aβ40 in the cerebrospinal fluid of the rabbit.

**Example 6 Plasminogen promotes degradation of human amyloid Aβ40 in cerebral homogenates of mouse models of Alzheimer's disease and normal mice**

Four B6SJLTg (APPSwFILon, PSEN1*M146L*L286V) 6799Vas/Mmjax (FAD) mice aged 11 weeks (stock number: 034840) (FAD for short) and four C57BL/6 (normal) mice aged 11 weeks were killed, the whole brain tissue was taken out, and cerebral homogenates were prepared as described in example 1 and transferred in EP tubes.

Eppendorf (EP) tubes were divided into ① a blank control group, ② a solvent control group, and ③ a plasminogen group, and 5 parallels were set for each group. 21.5 μL of normal saline, 4.6 μL of plasminogen solution (2 mg/mL), and 23.9 μL of cerebral homogenate of the mice were placed in each tube of the blank control group. 21.5 μL of Aβ40 (1.0 mg/mL, ChinaPeptides, 04010011521), 4.6 μL of solvent solution (10 mM sodium citrate, 2% arginine hydrochloride, and 3% mannitol, pH=7.4), and 23.9 μL of cerebral homogenate of the mice were placed in each tube of the solvent control group. 21.5 mL of Aβ40 (1.0 mg/mL), 4.6 μL of plasminogen solution (2 mg/mL), and 23.9 μL of cerebral homogenate of the mice were placed in each tube of the plasminogen group. Then, the materials in each tube were incubated at 37°C for 6 h, and 50 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). Each group of samples was electrophoresed as described in example 1, stained with Coomassie brilliant blue, then destained and scanned quantitatively.

The results show that in the cerebral homogenates of the mouse models of Alzheimer's disease, the amount of human amyloid Aβ40 in the plasminogen group is significantly less than that in the solvent control group, and the difference is extremely significant (*** indicates P<0.001); and in the cerebral homogenates of the normal mice, the amount of amyloid Aβ40 in the plasminogen group is significantly less than that in the solvent control group, and the difference is extremely significant (P=0.001) ( FIG. 6). It is indicated that plasminogen can effectively promote the degradation of human amyloid Aβ40 in the cerebral homogenates of the mouse models of Alzheimer's disease and the normal mice.

**Example 7 Plasminogen promotes degradation of human amyloid Aβ42 in cerebral homogenates of mouse models of Alzheimer's disease and normal mice**

Four B6SJLTg (APPSwF1Lon, PSEN1*M146L*L286V) 6799Vas/Mmjax (FAD) mice aged 11 weeks (stock number: 034840) (FAD for short) and four C57BL/6 (normal) mice aged 11 weeks were killed, the whole brain tissue was taken out, and cerebral homogenates were prepared as described in example 1 and transferred in EP tubes.

Eppendorf (EP) tubes were divided into ① a blank control group, ② a solvent control group, and ③ a plasminogen group, and 5 parallels were set for each group. 21.5 μL of normal saline, 4.6 μL of plasminogen solution (2 mg/mL), and 23.9 μL of cerebral homogenate of the mice were placed in each tube of the blank control group. 21.5 μL of Aβ42 (1.0 mg/mL, ChinaPeptides, 04010011526), 4.6 μL of solvent solution (10 mM sodium citrate, 2% arginine hydrochloride, and 3% mannitol, pH=7.4), and 23.9 μL of cerebral homogenate of the mice were placed in each tube of the solvent control group. 21.5 mL of Aβ42 (1.0 mg/mL), 4.6 μL of plasminogen solution (2 mg/mL), and 23.9 μL of cerebral homogenate of the mice were placed in each tube of the plasminogen group. Then, the materials in each tube were incubated at 37°C for 6 h, and 50 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). Each group of samples was electrophoresed as described in example 1, stained with Coomassie brilliant blue, then destained and scanned quantitatively.

The results show that in the cerebral homogenates of the mouse models of Alzheimer's disease, the amount of amyloid Aβ42 in the plasminogen group is less than that of the solvent control group, and the amounts of aggregates a, b, and c in the plasminogen group are all less than those in the solvent group, and the difference is extremely significant (* indicates P<0.05, and *** indicates P<0.001); and in the cerebral homogenates of the normal mice, the amount of amyloid Aβ42 in the plasminogen group is significantly less than that in the solvent control group, the difference is extremely significant (*** indicates P<0.001), the amounts of aggregates a, b, and c are all less than those in the solvent group, and the difference is extremely significant (*** indicates P<0.001) (FIG. 7). It is indicated that plasminogen can effectively promote the degradation of human amyloid Aβ42 in the cerebral homogenates of the mouse models of Alzheimer's disease and the normal mice.

**Example 8 Plasminogen promotes degradation of human Aβ42 in cerebral homogenates of mouse models of Alzheimer's disease and normal mice**

Four B6SJLTg (APPSwF1Lon, PSEN1*M146L*L286V) 6799Vas/Mmjax (FAD) mice aged 11 weeks (stock number: 034840) (FAD for short) and four C57BL/6 (normal) mice aged 11 weeks were killed, the whole brain tissue was taken out, and cerebral homogenates were prepared as described in example 1 and transferred in EP tubes.

Eppendorf (EP) tubes were divided into ① a blank control group, ② a solvent control group, and ③ a plasminogen group, and 5 parallels were set for each group. 21.5 μL of normal saline, 4.6 μL of plasminogen solution (2 mg/mL), and 23.9 μL of cerebral homogenate of the mice were placed in each tube of the blank control group. 21.5 μL of Aβ42 (1.0 mg/mL, ChinaPeptides, 04010011526), 4.6 μL of solvent solution (10 mM sodium citrate, 2% arginine hydrochloride, and 3% mannitol, pH=7.4), and 23.9 μL of cerebral homogenate of the mice were placed in each tube of the solvent control group. 21.5 mL of Aβ42 (1.0 mg/mL), 4.6 μL of plasminogen solution (2 mg/mL), and 23.9 μL of cerebral homogenate of the mice were placed in each tube of the plasminogen group. Then, the materials in each tube were incubated at 37°C for 6 h, and 50 μL of 0.1% trifluoroacetic acid solution was placed in each tube to terminate the reaction.

A 20% gel was prepared according to instructions of a Tris-Tricine-SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 1.5 h, and then at 100 V to the bottom of the gel. After electrophoresis was completed, the gel was peeled off and transferred to a PVDF membrane (GE, A29433753), and electrophoresis was performed at 15 V for 2 h. The transferred PVDF membrane was immersed in a blocking solution (a 5% skim emulsion) and blocked in a refrigerator at 4 °C overnight, washed four times with TBST (a 0.01 M Tris-NaCl buffer, pH=7.6), a mouse anti-human Aβ42 antibody (GenScript, 10842-1-AP) was added, the PVDF membrane was incubated at the room temperature for 3 h, and washed four times with TBST, a goat anti-mouse IgG (HRP) antibody (Abeam, ab6789) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that in the cerebral homogenates of the mouse models of Alzheimer's disease, the amount of amyloid Aβ42 in the plasminogen administration group is less than that of the solvent control group, and the amounts of aggregates in the plasminogen administration group are all less than those of the solvent group, and the difference is extremely significant (* indicates P<0.05); and in the cerebral homogenates of the normal mice, the amount of amyloid Aβ42 of the plasminogen group is significantly less than that of the solvent control group, the difference is extremely significant (*** indicates P<0.001), the amounts of the aggregates are all less than those in the solvent group, and the difference is extremely significant (*** indicates P<0.001) ( FIG. 8). It is indicated that plasminogen can effectively promote the degradation of human amyloid Aβ42 in the cerebral homogenates of the mouse models of Alzheimer's disease and the normal mice.

### Example 9 Plasminogen promotes degradation of Tau proteins in a cerebral homogenates of normal mice

Four C57BL/6J male mice aged 11-12 weeks with a body weight of 18-25 g were killed, the whole brain tissue was taken out and weighed, 1× PBS (pH=7.4, Thermo Fisher, 10010-031) was added at a rate of 150 mg tissue per 1 mL of PBS, the tissue was homogenized at 4°C (1 min/time, 3-4 times), the homogenate was centrifuged at 4 °C (at 12000 rpm for 20 min), and a supernatant cerebral homogenate was transferred to a new EP tube.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank group. 21.5 μL of normal saline, 4.6 μL of plasminogen solution (0.5 mg/mL), and 23.9 μL of cerebral homogenate of the mice were placed in each tube of the blank control group. 21.5 μL of Tau (1.0 mg/mL, customized expressed human Tau proteins, GenScript, UniProtKB - P10636-8), 4.6 μL of solvent solution, and 23.9 μL of cerebral homogenate of the mice were placed in each tube of the solvent control group. 21.5 μL of Tau (1.0 mg/mL), 4.6 μL of plasminogen solution (0.5 mg/mL), and 23.9 μL of cerebral homogenate of the mice were placed in each tube of the plasminogen group. Then, the materials in each tube were incubated at 37°C for 6 h, and 50 μL of 0.1% trifluoroacetic acid solution was placed in each tube to terminate the reaction.

A 10% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 45 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit-derived Tau protein antibody (Abeam, ab151559) was added, the PVDF membrane was incubated at the room temperature for 2 h, and washed four times with TBST, a goat anti-rabbit IgG (HRP) antibody (Abeam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

Tau proteins are the most abundant microtubule-associated proteins. Tau proteins are phosphate-containing proteins, and a Tau protein molecule in normal mature brain contains 2 or 3 phosphate groups. However, Tau proteins in the brain of a patient with Alzheimer's disease (senile dementia) are abnormally hyperphosphorylated, and each Tau protein molecule may contain 5 to 9 phosphate groups and lose normal biological functions[4].

The results show that in the cerebral homogenates of the normal mice, the amount of Tau proteins in the plasminogen group is significantly less than that in the solvent control group, and the difference is significant (* indicates P<0.05, ** indicates P<0.01, and *** indicates P<0.001) (FIG. 9). It is indicated that the plasminogen may promote the degradation of Tau proteins in the cerebral homogenates of the normal mice.

### Example 10 Plasminogen promotes degradation of Tau proteins in cerebral homogenates of mouse models of Alzheimer's disease

Four B6SJLTg (APPSwF1Lon, PSEN1*M146L*L286V) 6799Vas/Mmjax (FAD) mice aged 11 weeks (stock number: 034840) (FAD for short) were killed, the whole brain tissue was taken out, weighed, and placed into an Eppendorf (EP) tube, 1× PBS (pH=7.4, Thermo Fisher, 10010-031) was added at a rate of 150 mg tissue per 1 mL of PBS, the tissue was homogenized at 4°C (1 min/time, 3-4 times), the homogenate was centrifuged at 4 °C (at 12000 rpm for 20 min), and a supernatant cerebral homogenate was transferred to a new EP tube.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank group. 21.5 μL of normal saline, 4.6 μL

of plasminogen solution (0.5 mg/mL), and 23.9 $\mu$L of cerebral homogenate of the mice were placed in each tube of the blank control group. 21.5 $\mu$L of Tau (1.0 mg/mL, customized expressed human Tau proteins, GenScript, UniProtKB - P10636-8), 4.6 $\mu$L of solvent solution, and 23.9 $\mu$L of cerebral homogenate of the mice were placed in each tube of the solvent control group. 21.5 $\mu$L of Tau (1.0 mg/mL), 4.6 $\mu$L of plasminogen solution (0.5 mg/mL), and 23.9 $\mu$L of cerebral homogenate of the mice were placed in each tube of the plasminogen group. Then, the materials in each tube were incubated at 37°C for 6 h, and 50 $\mu$L of 0.1% trifluoroacetic acid solution was placed in each tube to terminate the reaction.

A 10% gel was prepared according to instructions of an SDS-PAGE gel preparation kit. A sample of each group was uniformly mixed with a 4 $\times$ loading buffer (TaKaRa, e2139) in a volume ratio of 3: 1, the mixture was heated at 100°C for 5 min, cooled, and centrifuged for 2 min, and 20 $\mu$L of sample was loaded. Electrophoresis was performed at 30 V for 45 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit-derived Tau protein antibody (Abeam, ab151559) was added, the PVDF membrane was incubated at the room temperature for 2 h, and washed four times with TBST, a goat anti-rabbit IgG (HRP) antibody (Abeam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that in the cerebral homogenates of the mouse models of Alzheimer's disease, the amount of Tau proteins in the plasminogen group is significantly less than that of the solvent control group, and the statistical difference is significant (* indicates P<0.05, ** indicates P<0.01) (FIG. 10). It is indicated that the plasminogen may promote the degradation of Tau proteins in the cerebral homogenates of the mouse models of Alzheimer's disease.

**Example 11 Plasminogen reduces the level of Tau proteins in the brain tissues of mouse models of Alzheimer's disease**

B6SJL-Tg (APPSwF1Lon, PSEN1*M146L*L286V) 6799Vas/Mmjax mice (purchased from Jackson lab, stock number: 034840) were backcrossed three times with C57BL/6J mice to breed offspring (B6-F3-FAD for short). Eighteen female B6-F3-FAD mice aged 20-25 weeks and nine female C57BL/6J mice aged 9 weeks were selected. The B6-F3-FAD mice were randomly divided into two groups, i.e. a solvent group and an administration group, according to the body weight and Y maze test results, 9 mice in each group. The nine C57BL/6J mice were taken as a blank control group. After grouping, a solvent solution was injected into each mouse of the blank control group and the solvent group via the tail vein at a dose of 5 mL/kg. Plasminogen was injected into each mouse of the administration group via the tail vein at a dose of 50 mg/kg for 28 consecutive days. 7 days after drug withdrawal, mice were randomly selected from each group and killed, the brain tissue was taken out and homogenized at 4°C, and a supernatant, i.e. a cerebral homogenate, was collected and subjected to a BCA protein assay for determining total protein and a Western blot assay.

A 10% gel was prepared according to instructions of an SDS-PAGE gel preparation kit. A sample of each group was uniformly mixed with a 4 $\times$ loading buffer (TaKaRa, e2139) in a volume ratio of 3: 1, the mixture was heated at 100°C for 5 min, cooled, and centrifuged for 2 min, and 20 $\mu$L of sample was loaded. Electrophoresis was performed at 30 V for 1.5 h, and then at 100 V to the bottom of the gel. After electrophoresis was completed, the gel was peeled off and transferred to a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-mouse Tau antibody (Abeam, ab 151559) was added, the PVDF membrane was incubated at the room temperature for 2 h, and washed four times with TBST, a goat anti-rabbit IgG (HRP) antibody (Abeam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that certain levels of Tau proteins having different molecular weights are present in cerebral homogenates of mice in the blank control group; the levels of Tau proteins having different molecular weights and the total Tau protein level in the brain tissues of mice in administration group are significantly lower than those in the solvent group, and statistical analysis P values between the two groups in the levels of Tau proteins having molecular weights of 35 kd, 35-40 kd, 40 kd, and 54 kd, and the total Tau protein level are 0.174, 0.0406, 0.052, 0.067, and 0.055, respectively (FIG. 11). It is indicated that the plasminogen can promote the degradation of Tau proteins in brain tissues of the mouse models of Alzheimer's disease.

**Example 12 Plasminogen promotes cleavage of Pro-BDNF in cerebral homogenates of normal mice**

Four male C57BL/6J mice aged 11-12 weeks with a body weight of 18-25 g were killed, the whole brain tissue was taken out and weighed, 1× PBS was (pH=7.4, Thermo Fisher, 10010-031) was added at a rate of 150 mg of tissue per 1 mL of PBS, the tissue was homogenized at 4 °C (3-4 times, 1 min/time) and then centrifuged at 4 °C (at 12000 rpm for 20 min), and a supernatant, i.e. a homogenate was transferred to a new EP tube.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank control group. 21.5 μL of normal saline, 4.6 μL of plasminogen solution (2 mg/mL), and 23.9 μL of cerebral homogenate of the mice were placed in each tube of the blank group. 21.5 μL of Pro-BDNF (1.0 mg/mL, customized expressed human Pro-BDNF, GenScript, UniProtKB - P23560), 4.6 μL of solvent solution, and 23.9 μL of cerebral homogenate of the mice were placed in each tube of the solvent control group. 21.5 μL of Pro-BDNF (1.0 mg/mL), 4.6 μL of plasminogen solution (2 mg/mL), and 23.9 μL of cerebral homogenate of the mice were placed in each tube of the plasminogen group. Then, the materials in each tube were incubated at 37 °C for 6 h, and 50 μL of 0.1% trifluoroacetic acid solution was placed in each tube to terminate the reaction.

A 12% gel was prepared according to instructions of an SDS-PAGE gel preparation kit. Each group of samples was electrophoresed as described in example 1, stained with Coomassie brilliant blue, then destained and scanned quantitatively.

Brain-derived neurotrophic factor (BDNF) is an alkaline protein having a molecular weight of 12.3 kDa, is composed of 119 amino acid residues, and contains three pairs of disulfide bonds. BDNF is present in the body in the form of dimer and synthesized in the form of a BDNF precursor (Pro-BDNF) that can be cleaved by enzymolysis to form mature BDNF. It has been reported in documents that Pro-BDNF has opposite effects to mature BDNF formed by cleaving Pro-BDNF. Pro-BDNF promotes apoptosis of nerve cells and reduces neural synaptic plasticity[5]. Mature BDNF and its receptors are widely found in the central nervous system, and play an important role in the survival, differentiation, and growth and development of neurons during the development of the central nervous system. Furthermore, they can prevent neuronal damage and apoptosis, improve the pathological state of neurons, promote biological effects, such as regeneration and differentiation, of injured neurons, and are also necessary for the survival and normal physiological functions of neurons in the mature central and peripheral nervous systems[6].

The results show that in the cerebral homogenates of the normal mice, the amount of Pro-BDNF in the plasminogen group is significantly lower than in the solvent control group, with a very significant difference (*** indicates $P<0.001$) (FIG. 12). It is suggested that the plasminogen can promote cleavage of Pro-BDNF in the cerebral homogenates of the normal mice.

**Example 13 Plasminogen promotes cleavage of Pro-BDNF in cerebral homogenates of normal mice to form mature BDNF**

Four male C57BL/6J mice aged 11-12 weeks with a body weight of 18-25 g were killed, the whole brain tissue was taken out and weighed, 1× PBS was (pH=7.4, Thermo Fisher, 10010-031) was added at a rate of 150 mg of tissue per 1 mL of PBS, the tissue was homogenized at 4 °C (3-4 times, 1 min/time) and then centrifuged at 4 °C (at 12000 rpm for 20 min), and a supernatant, i.e. a homogenate was transferred to a new EP tube.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank control group. 21.5 μL of normal saline, 4.6 μL of plasminogen solution (2 mg/mL), and 23.9 μL of cerebral homogenate of the mice were placed in each tube of the blank group. 21.5 μL of Pro-BDNF (1.0 mg/mL, customized expressed human Pro-BDNF, GenScript, UniProtKB - P23560), 4.6 μL of solvent solution, and 23.9 μL of cerebral homogenate of the mice were placed in each tube of the solvent control group. 21.5 μL of Pro-BDNF (1.0 mg/mL), 4.6 μL of plasminogen solution (2 mg/mL), and 23.9 μL of cerebral homogenate of the mice were placed in each tube of the plasminogen group. Then, the materials in each tube were incubated at 37 °C for 6 h, and 50 μL of 0.1% trifluoroacetic acid solution was placed in each tube to terminate the reaction.

A 12% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 45 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human BDNF antibody (Boster Biological Technology, PB9075) was added, the PVDF membrane was incubated at the room temperature for 3 h, and washed four times with TBST, a goat anti-rabbit IgG (HRP) antibody (Abeam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that in the cerebral homogenates of the normal mice, the amount of Pro-BDNF in the plasminogen group is significantly lower than that in the solvent control group, and the difference is very significant (** indicates $P<0.01$) (FIG. 13). It is suggested that the plasminogen can promote cleavage of Pro-BDNF in the cerebral homogenates of the normal mice.

## Example 14 Plasminogen promotes cleavage of Pro-BDNF in cerebral homogenates of mouse models of Parkinson's disease

Four C57BL/6J male mice aged 11-12 weeks and weighed 18-25 g were taken. Modeling was timed at 9: 00 am; 200 $\mu$L of normal saline was intraperitoneally injected into mice in a blank control group; 5 mg/mL MPTP solution was intraperitoneally injected into mice in a model group by 35 mg/kg per mouse for consecutive 5 days, and a Parkinson's disease model was established[6]. Preparation of the MPTP solution: 45 mg of MPTP (sigma, M0896) was dissolved into 9 mL normal saline solution to prepare the final concentration as 5 mg/mL. On the 6th day after the completion of modeling, all mice were subjected to open field test to identify the success of modeling. After all the mice were sacrificed, whole brains were taken and weighed; 1×PBS (Thermo Fisher, pH7.4; 10010-031) was added according to 150 mg tissues/mL PBS for homogenization at 4°C (1 min, 3-4 times); then centrifugation was conducted at 4 °C (12000 rpm, 20 min); supernatants, namely, cerebral homogenates were taken and placed in new EP tubes.

Eppendorf (EP) tubes were divided into ① a blank group, ② a blank control group, ③ a solvent control group, and ④ a plasminogen group, and 5 parallels were set for each group. 21.5 $\mu$L of normal saline, 4.6 $\mu$L of solvent solution (10 mM sodium citrate, 2% arginine hydrochloride, and 3% mannitol, pH=7.4), and 23.9 $\mu$L of cerebral homogenate of the mouse were placed in each tube of the blank group. 21.5 $\mu$L of normal saline, 4.6 $\mu$L of plasminogen solution (2 mg/mL), and 23.9 $\mu$L of cerebral homogenate of the mice were placed in each tube of the blank control group. 21.5 $\mu$L of Pro-BDNF solution (1.0 mg/mL, customized expressed human Pro-BDNF, GenScript, UniProtKB - P23560), 4.6 $\mu$L of solvent solution, and 23.9 $\mu$L of cerebral homogenate of the mice were placed in each tube of the solvent control group. 21.5 $\mu$L of Pro-BDNF solution (1.0 mg/mL), 4.6 $\mu$L of plasminogen solution (2 mg/mL), and 23.9 $\mu$L of cerebral

homogenate of the mice were placed in each tube of the plasminogen group. Then, the materials in each tube were incubated at 37 °C for 6 h, and 50 $\mu$L of 0.1% trifluoroacetic acid solution was placed in each tube to terminate the reaction.

A 12% gel was prepared according to instructions of an SDS-PAGE gel preparation kit. Each group of samples was electrophoresed as described in Example 1, stained with Coomassie brilliant blue, then destained and scanned quantitatively.

The results show that in the cerebral homogenates of the mouse models of Parkinson's disease, the amount of Pro-BDNF in the plasminogen group is significantly less than that in the solvent control group, and the difference is extremely significant (*** indicates $P<0.001$) (FIG. 14). It is suggested that the plasminogen can promote cleavage of Pro-BDNF in the cerebral homogenates of the mouse models of Parkinson's disease.

## Example 15 Plasminogen promotes cleavage of Pro-BDNF in cerebral homogenates of mouse models of Parkinson's disease to form mature BDNF

Eight C57BL/6J male mice aged 11-12 weeks with a body wight of 18-25 g were taken and weighed one day before modeling. The mice were randomly divided into two groups according to body weights with 4 in a blank control group and 4 in a model group. Modeling was timed at 9: 00 am; 200 $\mu$L of normal saline was intraperitoneally injected into mice in a blank control group; 5 mg/mL MPTP solution was intraperitoneally injected into mice in a model group by 35 mg/kg per mouse for consecutive 5 days, and a Parkinson's disease model was established[6]. Preparation of the MPTP solution: 45 mg of MPTP (sigma, M0896) was dissolved into 9 mL normal saline solution to prepare the final concentration as 5 mg/mL. On the 6th day after the completion of modeling, all mice were subjected to open field test to identify the success of modeling. After all the mice were sacrificed, whole brains were taken and weighed; 1×PBS (Thermo Fisher, pH7.4; 10010-031) was added according to 150 mg tissues/mL PBS for homogenization at 4 °C (1 min, 3-4 times); then centrifugation was conducted at 4 °C (12000 rpm, 20 min); supernatants, namely, cerebral homogenates were taken and placed in new EP tubes.

Eppendorf (EP) tubes were divided into ① a blank group, ② a blank control group, ③ a solvent control group, and ④ a plasminogen group, and 5 parallels were set for each group. 21.5 $\mu$L of normal saline, 4.6 $\mu$L of solvent solution (10 mM sodium citrate, 2% arginine hydrochloride, and 3% mannitol, pH=7.4), and 23.9 $\mu$L of cerebral homogenate of the mice were placed in each tube of the blank group. 21.5 $\mu$L of normal saline, 4.6 $\mu$L of plasminogen solution (2 mg/mL), and 23.9 $\mu$L of cerebral homogenate of the mice were placed in each tube of the blank control group. 21.5 $\mu$L of Pro-BDNF (1.0 mg/mL, customized expressed human Pro-BDNF, Gen-

Script, UniProtKB - P23560), 4.6 μL of solvent solution, and 23.9 μL of cerebral homogenate of the mice were placed in each tube of the solvent control group. 21.5 μL of Pro-BDNF (1.0 mg/mL), 4.6 μL of plasminogen solution (2 mg/mL), and 23.9 μL of cerebral homogenate of the mice were placed in each tube of the plasminogen group. Then, the materials in each tube were incubated at 37 °C for 6 h, and 50 μL of 0.1% trifluoroacetic acid solution was placed in each tube to terminate the reaction.

A 12% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 45 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human BDNF antibody (Boster Biological Technology, PB9075) was added, the PVDF membrane was incubated at the room temperature for 3 h, and washed four times with TBST, a goat anti-rabbit IgG (HRP) antibody (Abeam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), photographed by using a biomolecular imager, and quantitatively analyzed by using Image J.

The results show that in the cerebral homogenates of the mouse models of Parkinson's disease, the amount of Pro-BDNF in the plasminogen group is significantly less than that in the solvent control group, and the difference is extremely significant (** indicates P<0.05, and *** indicates P<0.001); and the amount of BDNF in the plasminogen group is significantly higher than that in the solvent control group, and the difference is extremely significant (FIG. 15). It is indicated that plasminogen can promote the cleavage of Pro-BDNF and formation of mature BDNF in the cerebral homogenates of the mouse models of Parkinson's disease.

**Example 16 Plasminogen promotes the cleavage of Pro-BDNF in cerebral homogenates of mouse models of Alzheimer's disease**

Four B6SJLTg (APPSwF1Lon, PSEN1*M146L*L286V) 6799Vas/Mmjax (FAD) mice aged 11 weeks (stock number: 034840) (FAD for short) were killed, the whole brain tissue was taken out, and cerebral homogenates were prepared as described in example 1 and transferred in EP tubes.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank group. 21.5 μL of normal saline, 4.6 μL of plasminogen solution (2 mg/mL), and 23.9 μL of cerebral homogenate of the mice were placed in each tube of the blank control group. 21.5 μL of Pro-BDNF (1.0 mg/mL, customized expressed human Pro-BDNF, GenScript, UniProtKB - P23560), 4.6 μL of solvent solution, and 23.9 μL of cerebral homogenate of the mice were placed in each tube of the solvent control group. 21.5 mL of Pro-BDNF (1.0 mg/mL), 4.6 μL of plasminogen solution (2 mg/mL), and 23.9 μL of cerebral homogenate of the mice were placed in each tube of the plasminogen group. Then, the materials in each tube were incubated at 37 °C for 6 h, and 50 μL of 0.1% trifluoroacetic acid solution was placed in each tube to terminate the reaction.

A 12% gel was prepared according to instructions of an SDS-PAGE gel preparation kit. Each group of samples was electrophoresed as described in Example 1, stained with Coomassie brilliant blue, then destained and scanned quantitatively.

The results show that in the cerebral homogenates of mouse models of Alzheimer's disease, the amount of Pro-BDNF in the plasminogen group is significantly less than that in the solvent control group, and the difference is extremely significant (*** indicates P<0.001) (FIG. 16). It is indicated that plasminogen can promote the cleavage of Pro-BDNF in the cerebral homogenates of the mouse models of Alzheimer's disease.

**Example 17 Plasminogen promotes cleavage of Pro-BDNF in cerebral homogenates of mouse models of Alzheimer's disease to form mature BDNF**

Four B6SJLTg (APPSwF1Lon, PSEN1*M146L*L286V) 6799Vas/Mmjax (FAD) mice aged 11 weeks (stock number: 034840) (FAD for short) were killed, the whole brain tissue was taken out, and cerebral homogenates were prepared as described in example 1 and transferred in EP tubes.

Eppendorf (EP) tubes were divided into ① a blank group, ② a blank control group, ③ a solvent control group, and ④ an administration group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank group. 21.5 μL of normal saline, 4.6 μL of plasminogen solution (2 mg/mL), and 23.9 μL of cerebral homogenate of the mice were placed in each tube of the blank control group. 21.5 μL of Pro-BDNF (1.0 mg/mL, customized expressed human Pro-BDNF, Gen-

Script, UniProtKB - P23560), 4.6 μL of solvent solution, and 23.9 μL of cerebral homogenate of the mice were placed in each tube of the solvent control group. 21.5 mL of Pro-BDNF (1.0 mg/mL), 4.6 μL of plasminogen solution (2 mg/mL), and 23.9 μL of cerebral homogenate of the mice were placed in each tube of the plasminogen group. Then, the materials in each tube were incubated at 37 °C for 6 h, and 50 μL of 0.1% trifluoroacetic acid solution was placed in each tube to terminate the reaction.

A 12% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 45 min and then at 100 V to the bottom of the gel. After electrophoresis was completed, the gel was peeled off and transferred to a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human BDNF antibody (Boster Biological Technology, PB9075) was added, the PVDF membrane was incubated at the room temperature for 3 h, and washed four times with TBST, a goat anti-rabbit IgG (HRP) antibody (Abeam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and optical densities of bands were quantitatively analyzed by using Image J.

The results show that in the cerebral homogenates of the mouse models of Alzheimer's disease, the amount of Pro-BDNF in the plasminogen group is significantly less than that in the solvent control group, and the difference is extremely significant (** indicates P<0.01, and *** indicates P<0.001); and the amount of BDNF in the plasminogen group is significantly higher than that in the solvent control group, and the difference is extremely significant (FIG. 17). It is indicated that plasminogen can promote the cleavage of Pro-BDNF and formation of mature BDNF in the cerebral homogenates of the mouse models of Alzheimer's disease.

**Example 18 Plasminogen promotes increased level of BDNF in hippocampus of mouse models of schizophrenia**

Before model construction, 30 male C57 mice were weighed, abnormal mice were excluded according to the body weight, and then all the mice were randomly divided into two groups, i.e. a blank control group and a model group, 8 mice in the blank control group and 22 mice in the model group. After completion of grouping, the mice in the blank control group were fed with a maintenance feed, and the mice in the model group were fed with a modeling feed containing 0.6% cuprizone (CPZ) (manufacturer: Shanghai Yuanye Biotechnology Co., Ltd.; Article No.: S30349) for consecutive 42 days to induce a schizophrenia model [7, 8]. After the completion of modeling, all the mice were tested in open field tests; and according to test results, the mice in the model group were divided into a solvent group with 11 mice and an administration group with 11 mice. After completion of grouping, all the mice started to be administered, denoted as the 1st day of administration; the mice in the blank control group were injected with a solvent (4% arginine + 2% glycine solution) via tail veins according to 0.1 ml/mouse per day; the mice in the solvent group were injected with a solvent via tail veins according to 0.1 ml/mouse per day; and the mice in the administration group were administrated with plasminogen via tail veins according to 1 ml/mouse per day for consecutive 35 days, and during administration, all the mice were fed with a normal maintenance feed. The mice were sacrificed on the 36th day, and the brain tissues were fixed in 10% formaldehyde solution, dehydrated and embedded. The fixed tissue sample was dehydrated with graded ethanol, cleared with xylene, and embedded in paraffin. Thicknesses of coronal sections of the brain tissues were 3 μm, and sections were dewaxed, rehydrated and then washed once. The sections were repaired with citric acid for 30 min, cooled at a room temperature for 10 min and rinsed gently with water. The sections were incubated with 3% hydrogen peroxide for 15 min, and the tissues were circled with a PAP pen. The sections were blocked with 10% goat serum (Vector laboratories, Inc., USA) for 1 h; and after the expiration of time, the sheep serum was discarded. A rabbit-derived anti-BDNF antibody (BosterBio, PB9075) was added for incubation overnight at 4°C, and the sections were washed with PBS for two times with 5 min each. A goat anti-rabbit IgG (HRP) antibody (Abcam) secondary antibody was added, and the sections were incubated at the room temperature for 1 h, and washed twice with PBS for 5 min each time. The sections were developed according to a DAB kit (Vector laboratories, Inc., USA), washed with water for 3 times, then counterstained with hematoxylin for 30 s, returned to blue with running water for 5 min and then washed with PBS once. The sections were gradiently dehydrated and cleared, blocked and observed and photographed under a 400X light microscope; and photographs were analyzed for an optical density of positive staining using an Imaging-Pro software.

Mature BDNF and its receptors are widely found in the central nervous system, and play an important role in the survival, differentiation, and growth and development of neurons during the development of the central nervous system. Furthermore, they can prevent neuronal damage and apoptosis, improve the pathological state of neurons, promote biological effects, such as regeneration and differentiation, of injured neurons, and are also necessary for the survival and normal physiological functions of neurons in the mature central and peripheral nervous systems[5].

The results show that the hippocampus of the mice in the blank control group (FIG. 18A) has a certain level of BDNF (indicated by an arrow); the level of BDNF in the hippocampus of the mice in the solvent group (FIG. 18B) is increased; and the level of BDNF in the hippocampus of the mice in the administration group is significantly higher than that in the solvent group (FIG. 18C), and the statistical difference is close to significance (FIG. 18D) (P=0.095). It is suggested that the plasminogen can increase the level of BDNF in the hippocampus of the mouse models of schizophrenia.

**Example 19 Plasminogen promotes expression of BDNF in hippocampus of mouse models of Alzheimer's disease**

Before model construction, twenty-three male C57 mice aged 24 weeks were weighed, abnormal mice were excluded according to the body weight, and then all the mice were randomly divided into two groups, i.e. a blank control group and a model group, 7 mice in the blank control group and 16 mice in the model group. All the mice were anesthetized; and after granular cell layers localized in the hippocampus were grouped according to a mouse stereotaxic atlas (localized according to coordinates of bregma: AP-2.0 mm, ML $\pm$ 1.5 mm, DV 2.0 mm), each mouse was subjected to micro-injection in both sides slowly; the mice in the model group were injected with an A$\beta$1-42 oligomer solution; and the mice in the blank control group were injected with the same volume of PBS solution at an injection rate of 0.4 $\mu$L/min with an injection volume of 2 $\mu$L. After injection, a syringe was slowly withdrawn after standing for 5 min to establish an Alzheimer model[3]. Preparation of the A$\beta$1-42 oligomer solution (10 $\mu$M): $\beta$-Amyloid (1-42) (Article Number: D2650; manufacturer: Sigma) was added to cold hexafluoroisopropanol to form a solution at a concentration of 1 mg/mL, and the solution was placed at the room temperature for 3 days, subpackaged at a volume of 45 $\mu$L/tube, i.e. 10 nmol/mL, placed in a fume hood overnight, dried in a drying oven at 25 °C for 1 hour, and preserved at -80°C. When used, 10 $\mu$L of dimethyl sulfoxide solution was placed in each tube to redissolve, before injection, 990 $\mu$L of sterile PBS was placed in each tube, and the mixture was placed at 4 °C for 24 hours and then used. After 28 days of brain stereotaxic injection, all the mice were weighed and tested by a Y maze, abnormal mice of the blank control group and the model group were excluded according to the test results. The mice of the model group were randomly divided into two groups, i.e. a solvent group and an administration group, 6 mice in the solvent group, 7 mice in the administration group, and 6 mice in the blank control group. Drugs were administered to the mice of the solvent group and the administration group, and the day on which administration was started was denoted as the 1st day. Plasminogen was injected into each mouse of the administration group via the tail vein at a dose of 1 mg/0.1 mL/day, a solvent solution (4% arginine and 2% glycine) was injected into each mouse of the solvent group via the tail vein at a dose of 0.1 mL/day, administration was performed for 28 consecutive days, and no drug was administered to the mice of the blank control group. On the 29th day, the mice were killed, and the brain tissue was taken out and fixed in 10% formaldehyde for 24-48 h. The fixed brain tissue was dehydrated with graded ethanol, cleared with xylene, and embedded in paraffin. The substantia nigra in a slice with a thickness of 4 $\mu$m was positioned, and the slice was subjected to deparaffinage and rehydration, and then washed once with water. The slice was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse BDNF antibody (BosterBio, PB9075) was added dropwise, and the slice was incubated at 4 °C overnight, and washed twice with 0.01 M PBS for 5 min each time. A goat anti-rabbit IgG (HRP) antibody (Abcam) secondary antibody was added, and the slice was incubated at the room temperature for 1 h, and washed twice with 0.01 M PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 200$\times$ optical microscope.

The results show that a certain level of BDNF (indicated by arrows) is expressed in the hippocampus of the mice in the blank control group (FIG. 19A); the expression of BDNF in the hippocampus of the mice in the solvent group (FIG. 19B) is significantly lower than that of the mice in the blank control group; the expression of BDNF in the hippocampus of the mice in the administration group (FIG. 19C) is significantly higher than that in the solvent group, and the statistical difference is significant (* indicates P<0.05) (FIG. 19D). It is indicated that plasminogen can promote the expression of BDNF in the hippocampus of the mouse models of Alzheimer's disease.

**Example 20 Plasminogen promotes formation of mature NGF in brain tissues of mouse models of spinal muscular atrophy (SMA)**

SMN1 genes of FVB. Cg-Grm7Tg (SMN2) 89Ahmb SmnItm1MsdTg (SMN2*delta7) 4299Ahmb/J gene mutant mice (hereinafter referred to as SMN $\Delta$7 SMA mice) are homozygously mutated and express human SMN2 genes; and the clinical and pathological manifestations of the mice are similar to those of human SMA.

Donor mice were purchased from Jackson Laboratories, USA (Pedigree Number: 005025).

Seven SMN Δ7 SMA mice born for 3 days were taken, wherein four mice were grouped into a solvent group, intraperitoneally injected with 6 μL of bovine serum albumin solution (5 mg/ml) once daily in the morning and the afternoon of the first 9 days, and intraperitoneally injected with 6 μL of bovine serum albumin solution (10 mg/ml) once in the following days; three mice were grouped into an administration group, intraperitoneally injected with plasminogen by 30 μg/6 μl once daily in the morning and the afternoon for the first 9 days, and intraperitoneally injected with the plasminogen by 60 μg/6 μL once daily for the following days; and four wild-type mice were taken as a blank control group, intraperitoneally injected with 6 μL of bovine serum albumin solution (5 mg/ml) once daily in the morning and afternoon for the first 9 days, and intraperitoneally injected with 6 μL of bovine serum albumin solution (10 mg/ml) once daily on the 10th day. On the 12th day, the mice were sacrificed to take hindlimb muscle tissue, and tissue homogenates were prepared for a Western blot test of an NGF protein. A 12% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 45 min, and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4 °C overnight, washed four times with TBST (a 0.01 M Tris-NaCl buffer, pH=7.6), a rabbit anti-mouse NGF antibody (Abeam, ab52918) was added, the PVDF membrane was incubated at the room temperature for 2 h, and washed four times with TBST, a goat anti-rabbit IgG (HRP) antibody (Abeam, ab6721) secondary antibody was added, and the PVDF membrane was incubated at the room temperature for 1 h, washed four times with TBST, placed on a clean imaging plate, developed with Immobilon Western HRP Substrate (MILLIPORE, WBKLS0100) and photographed by using a biomolecular imager, and an optical density value of each band is obtained by using Image J for quantitative analysis.

Nerve growth factor (NGF) is an important member of the neurotrophic factor family. It is synthesized in vivo in the form of precursor, and includes signal peptide, leader peptide, and mature peptide. Researches have reported that the nerve growth factor (NGF) precursor (Pro-NGF) has opposite effects to NGF formed by cleaving Pro-NGF. Pro-NGF can promote apoptosis of nerve cells. Mature NGF participates in the regulation of growth, development, differentiation, survival, post-injury repair, and other processes of nerve cells, and also plays an important role in regulating the functional expression of central and peripheral neurons[7]. NGF/Pro-NGF ratio = NGF optical density (OD)/Pro-NGF optical density (OD) value.

The results show that the brain tissues of the mice in the blank control group have a certain NGF/ProNGF ratio; and the NGF/Pro-NGF ratio of the brain tissues of the mice in the administration group is significantly higher than that of the mice in the solvent group, and the statistical difference is very significant (*** indicates P<0.001) (FIG. 20). It is suggested that the plasminogen can promote transformation of ProNGF to form NGF and promote formation of the mature NGF in the SMA mouse models.

## Example 21 Plasminogen promotes cleavage of Pro-NGF and formation of mature NGF in cerebral homogenates of normal mice

Four male C57BL/6J mice aged 11-12 weeks with a body weight of 18-25 g were killed, the whole brain tissue was taken out and weighed, 1× PBS was (pH=7.4, Thermo Fisher, 10010-031) was added at a rate of 150 mg of tissue per 1 mL of PBS, the tissue was homogenized at 4 °C (3-4 times, 1 min/time) and then centrifuged at 4 °C (at 12000 rpm for 20 min), and a supernatant, i.e. a homogenate was transferred to a new EP tube.

Eppendorf (EP) tubes were divided into ① a blank group, ② a blank control group, ③ a solvent control group, and ④a plasminogen group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank group. 21.5 μL of normal saline, 4.6 μL of plasminogen solution (2 mg/mL), and 23.9 μL of cerebral homogenate of the mice were placed in each tube of the blank control group. 21.5 μL of Pro-NGF (1.0 mg/mL, customized expressed human Pro-NGF, GenScript, sequence source: UniProtKB - P01138), 4.6 μL of solvent solution, and 23.9 μL of cerebral homogenate of the mice were placed in each tube of the solvent control group. 21.5 μL of Pro-NGF (1.0 mg/mL), 4.6 μL of plasminogen solution (2 mg/mL), and 23.9 μL of cerebral homogenate of the mouse were placed in each tube of the plasminogen group. Then, the materials in each tube were incubated at 37 °C for 6 h, and 50 μL of 0.1% trifluoroacetic acid solution was placed in each tube to terminate the reaction.

A 15% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 30 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human NGF antibody (Abeam, ab52918) was added, the PVDF membrane was incubated at the room temperature for 2 h, and washed four times with TBST, a goat anti-rabbit IgG (HRP) antibody (Abeam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and optical densities of bands were quantitatively analyzed by using Image J.

NGF is an important member in the neurotrophin family. It is synthesized in vivo in the form of precursor, and includes signal peptide, leader peptide, and mature peptide. Researches have reported that the nerve growth factor (NGF) precursor (ProNGF) has opposite effects to NGF formed by cleaving ProNGF. ProNGF can promote apoptosis of nerve cells[8]. Mature NGF participates in the regulation of growth, development, differentiation, survival, post-injury repair, and other processes of nerve cells, and also plays an important role in regulating the functional expression of central and peripheral neurons[9].

The results show that in the cerebral homogenates of normal mice, the amount of Pro-NGF in the plasminogen group is significantly less than that in the solvent control group, and the difference is extremely significant (* indicates P<0.05, and *** indicates P<0.001); and the amount of NGF in the plasminogen administration group is significantly higher than that in the solvent control group, and the difference is extremely significant (FIG. 21). It is suggested that the plasminogen can promote cleavage of Pro-NGF and formation of mature NGF in the cerebral homogenates of the normal mice.

**Example 22 Plasminogen promotes cleavage of Pro-NGF in cerebral homogenates of mouse models of Alzheimer's disease to form mature NGF**

Four B6SJLTg (APPSwFlLon, PSEN1*M146L*L286V) 6799Vas/Mmjax (FAD) mice aged 11 weeks (stock number: 034840) (FAD for short) were killed, the whole brain tissue was taken out, and cerebral homogenates were prepared as described in example 1 and transferred in EP tubes.

Eppendorf (EP) tubes were divided into ① a blank group, ② a blank control group, ③ a solvent control group, and ④ a plasminogen group, and 5 parallels were set for each group. In the blank control group, 21.5 μL of normal saline, 4.6 μL of plasminogen solution (2 mg/mL) and 23.9 μL of cerebral homogenate of the mice were added; in the solvent control group, 21.5 μL of Pro-

NGF (1.0 mg/mL, customized expressed human Pro-NGF, GenScript, UniProtKB-P01138), 4.6 μL of solvent solution and 23.9 μL of cerebral homogenate of the mice were added; and in the plasminogen group, 21 μL of Pro-NGF (1.0 mg/mL), 4.6 μL of plasminogen solution (2 mg/mL) and 23.9 μL of cerebral homogenate of the mice were added. Then, the materials in each tube were incubated at 37 °C for 6 h, and 50 μL of 0.1% trifluoroacetic acid solution was placed in each tube to terminate the reaction.

A 15% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 30 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human NGF antibody (Abeam, ab52918) was added, the PVDF membrane was incubated at the room temperature for 2 h, and washed four times with TBST, a goat anti-rabbit IgG (HRP) antibody (Abeam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and optical densities of bands were quantitatively analyzed by using Image J.

The results show that in the cerebral homogenates of the mouse models of Alzheimer's disease, the amount of Pro-NGF in the plasminogen group is significantly less than that in the solvent control group, and the difference is extremely significant (*** indicates P<0.001); the amount of NGF in the plasminogen group is significantly greater than that in the solvent control group, and the difference is significant (FIG. 22). It is indicated that plasminogen can promote the cleavage of Pro-NGF and formation of mature NGF in the cerebral homogenates of the mouse models of Alzheimer's disease.

**Example 23 Plasminogen promotes degradation of SOD-1 protein in cerebral homogenates of normal mice**

Four C57BL/6 (normal) mice aged 11 weeks were selected; after the mice were sacrificed, whole brain tissues were taken; and supernatants, namely, cerebral homogenates were prepared as described in example 1 and placed in EP tubes for standby.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank group. 21.5 μL of normal saline, 4.6 μL of plasminogen solution (2 mg/mL), and 23.9 μL of cerebral homogenate of the mice were placed in each tube of the blank control group. 21.5 μL of SOD-1 protein (1.0 mg/mL, customized expressed human SOD-1, Gen-Script, UniProtKB - P00441), 4.6 μL of solvent solution, and 23.9 μL of cerebral homogenate of the mice were placed in each tube of the solvent control group. 21.5 μL of SOD-1 protein (1.0 mg/mL), 4.6 μL of plasminogen solution (2 mg/mL), and 23.9 μL of cerebral homogenate of the mice were placed in each tube of the plasminogen group. Then, the materials in each tube were incubated at 37 °C for 6 h, and 50 μL of 0.1% trifluoroacetic acid solution was placed in each tube to terminate the reaction.

A 12% gel was prepared according to instructions of an SDS-PAGE gel preparation kit. Each group of samples was electrophoresed as described in Example 1, stained with Coomassie brilliant blue, then destained and scanned quantitatively.

Superoxide dismutase-1 (SOD-1) is a metalloprotein that binds to copper ions and zinc ions and plays an important role in transformation of hyper-reactivity superoxides of reactive oxygen species to oxygen molecules and hydrogen peroxide. It is known that in some patients with amyotrophic lateral sclerosis (ALS) as a kind of refractory neurological diseases, SOD-1 genes are mutated, and SOD-1 misfolded in an abnormal stereo structure can accumulate in motor nerves of a spinal cord. In addition, highly toxic misfolded SOD-1 has also been detected in cerebrospinal fluids from patients with unexplained sporadic ALS without mutation in SOD-1 encoding genes. That is, structural abnormalities of SOD-1 may be related to pathogenesis of ALS[10].

The results show that the amount of SOD-1 in the plasminogen group is significantly lower than that in the solvent control group in the cerebral homogenates of the normal mice (*** indicates P<0.001) (FIG. 23). It is indicated that the plasminogen may effectively promote the degradation of the SOD-1 protein in the cerebral homogenates of the normal mice.

**Example 24 Plasminogen promotes degradation of SOD-1 protein in cerebral homogenates of mouse models of amyotrophic lateral sclerosis (ALS)**

Four B6. Cg-Tg (SOD1-G93A) 1Gur/J transgenic male mice aged 8 weeks (abbreviated as SOD1-G93A transgenic mice) were selected. After sacrifice, the whole brain tissues were taken, and the supernatant, namely, the cerebral homogenate was prepared as described in Example 6 in EP tubes for later use.

Eppendorf (EP) tubes were divided into ① a blank group, ② a blank control group, ③ a solvent control group, and ④ an administration group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank group. 21.5 μL of normal saline, 4.6 μL of plasminogen solution (2 mg/mL), and 23.9 μL of cerebral homogenate of the mice were placed in each tube of the blank control group. 21.5 μL of SOD-1 protein (1.0 mg/mL, customized expressed human SOD-1, Gen-Script, UniProtKB - P00441), 4.6 μL of solvent solution, and 23.9 μL of cerebral homogenate of the mice were placed in each tube of the solvent control group. 21.5 μL of SOD-1 protein (1.0 mg/mL), 4.6 μL of plasminogen solution (2 mg/mL), and 23.9 μL of cerebral homogenate of the mice were placed in each tube of the plasminogen group. Then, the materials in each tube were incubated at 37 °C for 6 h, and 50 μL of 0.1% trifluoroacetic acid solution was placed in each tube to terminate the reaction.

A 12% gel was prepared according to instructions of an SDS-PAGE gel preparation kit. Each group of samples was electrophoresed as described in example 1, stained with Coomassie brilliant blue, then destained and scanned quantitatively.

SOD1-G93A transgenic mice are a widely used model mice for researching Amyotrophiclateral sclerosis (ALS)[11].

The results show that in the cerebral homogenates of the SOD1-G93A transgenic mice, the amount of the SOD-1 protein in the plasminogen group is significantly lower than that in the solvent control group, with a very significant difference (*** indicates P<0.001) (FIG. 24). It is indicated that the plasminogen may effectively promote the degradation of the SOD-1 protein in the cerebral homogenates in the mouse models of ALS.

**Example 25 Plasminogen promotes degradation of SOD-1 protein in cerebral homogenates of normal mice**

Four C57BL/6 (normal) mice aged 11 weeks were selected; after the mice were sacrificed, whole brain tissues were taken; and supernatants, namely, cerebral homogenates were prepared as described in example 1 and placed in EP tubes for standby.

Eppendorf (EP) tubes were divided into ① a blank group, ② a blank control group, ③ a solvent control group, and ④ a plasminogen group, and 5 parallels were set for each group. 21.5 μL of normal saline, 4.6 μL of solvent solution (10 mM sodium citrate, 2% arginine hydrochloride, and 3% mannitol, pH=7.4), and 23.9 μL of cerebral homogenate of the mice were placed in each tube of the blank group. 21.5 μL of normal saline, 4.6 μL of plasminogen solution (2 mg/mL), and 23.9 μL of cer-

ebral homogenate of the mice were placed in each tube of the blank control group. 21.5 µL of SOD-1 protein (1.0 mg/mL, customized expressed human SOD-1, Gen-Script, UniProtKB - P00441), 4.6 µL of solvent solution, and 23.9 µL of cerebral homogenate of the mice were placed in each tube of the solvent control group. 21.5 µL of SOD-1 protein (1.0 mg/mL), 4.6 µL of plasminogen solution (2 mg/mL), and 23.9 µL of cerebral homogenate of the mice were placed in each tube of the plasminogen group. Then, the materials in each tube were incubated at 37 °C for 6 h, and 50 µL of 0.1% trifluoroacetic acid solution was placed in each tube to terminate the reaction.

A 15% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100°C for 5 min, cooled, and centrifuged for 2 min, and 20 µL of sample was loaded. Electrophoresis was performed at 30 V for 30 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human SOD-1 antibody (BOSTER Biological Technology, PB0453) was added, the PVDF membrane was incubated at the room temperature for 3 h, and washed four times with TBST, a goat anti-rabbit IgG (HRP) antibody (Abeam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and optical densities of bands were quantitatively analyzed by using Image J.

The results show that in the cerebral homogenates of the normal mice, the amount of the SOD-1 protein in the plasminogen group is significantly lower than that in the solvent control group, and the difference is very significant (*** indicates P<0.001) (FIG. 25). It is indicated that the plasminogen may effectively promote the degradation of the SOD-1 protein in the cerebral homogenates of the normal mice.

**Example 26 Plasminogen promotes degradation of SOD-1 protein in cerebral homogenates of mouse models of amyotrophic lateral sclerosis (ALS)**

Four B6. Cg-Tg (SOD1-G93A) 1Gur/J transgenic male mice (referred to as SOD1-G93A transgenic mice) aged 8 weeks were taken; after the mice were sacrificed, whole brain tissues were taken; and supernatants, namely, cerebral homogenates were prepared as described in example 1 and placed in EP tubes for standby.

Eppendorf (EP) tubes were divided into ① a blank group, ② a blank control group, ③ a solvent control group, and ④ a plasminogen group, and 5 parallels were set for each group. 21.5 µL of normal saline, 4.6 µL of solvent solution (10 mM sodium citrate, 2% arginine hydrochloride, and 3% mannitol, pH=7.4), and 23.9 µL of cerebral homogenate of the mice were placed in each tube of the blank group. 21.5 µL of normal saline, 4.6 µL of plasminogen solution (2 mg/mL), and 23.9 µL of cerebral homogenate of the mice were placed in each tube of the blank control group. 21.5 µL of SOD-1 protein (1.0 mg/mL, customized expressed human SOD-1, Gen-Script, UniProtKB - P00441), 4.6 µL of solvent solution, and 23.9 µL of cerebral homogenate of the mice were placed in each tube of the solvent control group. 21.5 µL of SOD-1 protein (1.0 mg/mL), 4.6 µL of plasminogen solution (2 mg/mL), and 23.9 µL of cerebral homogenate of the mice were placed in each tube of the plasminogen group. Then, the materials in each tube were incubated at 37 °C for 6 h, and 50 µL of 0.1% trifluoroacetic acid solution was placed in each tube to terminate the reaction.

A 15% gel was prepared according to instructions of an SDS-PAGE gel preparation kit. A sample of each group was uniformly mixed with a 4× loading buffer (TaKaRa, e2139) in a volume ratio of 3: 1, the mixture was heated at 100 °C for 5 min, cooled, and centrifuged for 2 min, and 20 µL of sample was loaded. Electrophoresis was performed at 30 V for 30 min and then at 100 V to the bottom of the gel. After electrophoresis was completed, the gel was peeled off and activated onto a PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human SOD-1 antibody (Boster Biological Technology, PB0453) was added, the PVDF membrane was incubated at the room temperature for 3 h, and washed four times with TBST, a goat anti-rabbit IgG (HRP) antibody (Abeam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and optical densities of bands were quantitatively analyzed by using Image J.

The results show that in the cerebral homogenates of the SOD1-G93A transgenic mice, the amount of the SOD-1 protein in the plasminogen group is significantly lower than that in the solvent control group, with a very significant difference (*** indicates P<0.001) (FIG. 26). It is indicated that the plasminogen may effectively promote the degradation of the SOD-1 protein in the cerebral homogenates of the SOD1-G93A transgenic mice.

References

[1] Won-Seok Choi, et al. Conditional deletion of Ndufs4 in dopaminergic neurons promotes Parkinson's disease-like nonmotor symptoms without loss of dopamine neurons. Scientific Reports.

[2] KALIA L V, KALIA S K. Alpha- Synuclein and Lewy pathology in Parkinson's disease[J]. Curr Opin Neurol, 2015, 28 (4): 375-381.

[3] Selkoe D J. Alzheimer's disease: genes, proteins, and therapy [J]. Physiol. Rev, 2001, 81 (2): 741-766.

[4] Naseri NN, Wang H, Guo J, Sharma M, Luo W. The complexity of tau in Alzheimer's disease. Neurosci Lett. 2019 Jul 13; 705: 183-194.

[5] Gray K, Ellis V. Activation of pro-BDNF by the pericellular serine protease plasmin [J]. Febs Letters, 2008, 582 (6): 907-910.

[6] Kowianski, Przemys?aw, Lietzau G, Czuba E, et al. BDNF: A Key Factor with Multipotent Impact on Brain Signaling and Synaptic Plasticity [J]. Cellular & Molecular Neurobiology, 2017.

[7] Aloe L, Rocco M L, Bianchi P, et al. Nerve growth factor: from the early discoveries to the potential clinical use [J]. Journal of Translational Medicine, 2012, 10 (1).

[8] Aloe L, Rocco M L, Bianchi P, et al. Nerve growth factor: from the early discoveries to the potential clinical use [J]. Journal of Translational Medicine, 2012, 10 (1).

[9] Lewin G R, Lechner S G, Smith E S J. Nerve Growth Factor and Nociception: From Experimental Embryology to New Analgesic Therapy[J]. 2014.

[10] Anzai I, Tokuda E, Handa S, Misawa H, Akiyama S, Furukawa Y. Oxidative misfolding of Cu/Zn-superoxide dismutase triggered by non-canonical intramolecular disulfide formation. Free Radic Biol Med. 2020 Feb 1; 147:187-199.

[11] Zhao J, Boyd AW, Bartlett PF. The identification of a novel isoform of EphA4 and ITS expression in SOD1G93A mice. Neuroscience. 2017 Apr 7; 347:11-21.

**Claims**

1. A method for promoting the degradation of a misfolded protein and an aggregate thereof, comprising: administering a therapeutically effective amount of one or more compounds to a subject, the one or more compounds being selected from: plasminogen activation pathway components, a compound capable of directly activating plasminogen or indirectly activating plasminogen by activating plasminogen activation pathway upstream components, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.

2. The method according to claim 1, wherein the plasminogen activation pathway component is selected from plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, micro-plasmin, delta-plasminogen, delta-plasmin, a plasminogen activator, tPA, and uPA.

3. The method according to claim 1, wherein the antagonist of the fibrinolysis inhibitor is an inhibitor of PAI-1, a complement C1 inhibitor, $\alpha$2 antiplasmin or an $\alpha$2 macroglobulin, such as an antibody.

4. The method according to any one of claims 1-3, wherein the compound promotes the degradation of one or more misfolded proteins and aggregates thereof selected from: human amyloid A$\beta$40, human amyloid A$\beta$42, $\alpha$-synuclein, a Tau protein, an SOD-1 protein, polyglutamine, neuroserpin (NSP), a cystic fibrosis transmembrane conductance regulator, $\alpha$1-antitrypsin, a Parkin protein, crystallin, transthyretin, a short-chain acyl-CoA dehydrogenase variant, a low density lipoprotein receptor, huntingtin, a neurofilament protein, peripherin, an $\alpha$-internexin, islet amyloid polypeptide, $\beta$2-microglobulin, a serum amyloid A protein, an immunoglobulin light chain, human lysozyme, $\alpha$-lactalbumin, prothymosin $\alpha$, apolipoprotein E and apolipoprotein J.

5. The method according to any one of claims 1-3, wherein the compound promotes cleavage of Pro-BDNF to mature BDNF, or promotes cleavage of Pro-NGF to mature NGF.

6. The method according to any one of claims 1 to 5, wherein the compound is plasminogen.

7. The method according to any one of claims 1 to 6, wherein the plasminogen is human full-length plasminogen or a conservatively substituted variant thereof.

8. The method according to any one of claims 1 to 6, wherein the plasminogen has at least 75%, 80%, 85%, 90%, 95%, 96%, 97%, 98% or 99% sequence identity with sequence 2 and still has the lysine binding activity or the proteolytic activity of plasminogen.

9. The method according to any one of claims 1 to 6, wherein the plasminogen is a protein containing an amino acid sequence that has at least 80%, 90%, 95%, 96%, 97%, 98% or 99% amino acid sequence identity with sequence 14 and still having the proteolytic activity of plasminogen.

10. The method according to any one of claims 1 to 6, wherein the plasminogen is selected from Glu-plasminogen, Lys-plasminogen, mini-plasminogen, micro-plasminogen, delta-plasminogen, and variants thereof that retain the proteolytic activity of plasminogen.

11. The method according to any one of claims 1 to 6, wherein the plasminogen contains an amino acid sequence shown as sequence 2, 6, 8, 10 or 12, or contains a conservatively substituted variant of the amino acid sequences shown as sequence 2, 6, 8, 10 or 12.

12. The method according to any one of claims 1 to 11, wherein the compound is used in combination with one or more other treatment methods or drugs.

13. The method according to claim 12, wherein the other treatment methods comprise a cell therapy (comprising a stem cell therapy), a support therapy, and a physical therapy.

14. The method according to claim 12, wherein the one or more other drugs are drugs for treating the Alzheimer's disease, amyotrophic lateral sclerosis (ALS), or the Parkinson's disease.

15. The method according to any one of claims 1 to 14, wherein the compound is administered by nasal inhalation, aerosol inhalation, nasal drops, eye drops, ear drops, an intravenous method, an intraperitoneal method, a subcutaneous method, an intracranial method, an intrathecal method, an intraarterial method (e.g. via the carotid artery) or an intramuscular method.

Figure 1

Figure 2

Figure 3

Figure 4

| Lane | Quantitative scanning (%) |
|------|---------------------------|
| 1 | 0 |
| 2 | 100 |
| 3 | 75.81 |
| 4 | 0 |

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

Figure 24

Figure 25

Figure 26

## INTERNATIONAL SEARCH REPORT

| International application No. |
|---|
| **PCT/CN2021/082720** |

**A. CLASSIFICATION OF SUBJECT MATTER**

A61K 38/43(2006.01)i; A61K 38/49(2006.01)i; A61P 25/28(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, SIPOABS, DWPI, CNKI, Web of Science, Elsevier Science, 超星读秀, SUPERSTAR DUXIU: 纤维蛋白溶解酶原, 纤溶酶原, 突触核蛋白, 淀粉样蛋白, Tau, BDNF, NGF, SOD-1, 帕金森病, 阿尔兹海默症, 精神分裂症, 脊髓性肌萎缩, 肌萎缩性侧索硬化症, plasminogen, synuclein, amyloid protein, Tau protein, brain-derived neurotrophic factor, nerve growth factor, superoxide dismutase-1, Parkinson's disease, Alzheimer's disease, schizophrenia, Spinal Muscular Atrophy, Amyotrophic Lateral Sclerosis

**C. DOCUMENTS CONSIDERED TO BE RELEVANT**

| Category* | Citation of document, with indication, where appropriate, of the relevant passages | Relevant to claim No. |
|---|---|---|
| X | WO 0158476 A2 (THE EUROPEAN MOLECULAR BIOLOGY LABORATORY et al.) 16 August 2001 (2001-08-16) see claims 1-12 and 34-36, and description, page 8, paragraph 4, page 17, paragraph 5 and page 21, paragraph 5 | 1-4, 6-15 |
| X | US 2014186423 A1 (GELFAND MATHEW) 03 July 2014 (2014-07-03) see claims 1, 2 and 15-17 | 1, 2, 4, 12-15 |
| X | WO 03071267 A1 (VANDERBILT UNIVERSITY et al.) 28 August 2003 (2003-08-28) see the abstract, and claim 1 | 1, 3, 4 |
| X | US 6471960 B1 (RUTGERS, THE STATE UNIVERSITY) 29 October 2002 (2002-10-29) see claims 1 and 2 | 1, 2, 4 |
| X | WO 0162799 A2 (UNIVERSITAIR MEDISCH CENTRUM UTRECHT et al.) 30 August 2001 (2001-08-30) see claims 1, 7 and 8 | 1, 2, 4 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **21 May 2021** | **09 June 2021** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/082720** |

**C.    DOCUMENTS CONSIDERED TO BE RELEVANT**

| Category* | Citation of document, with indication, where appropriate, of the relevant passages | Relevant to claim No. |
| --- | --- | --- |
| X | ANGELUCCI Francesco et al. "Amyloid Beta Soluble Forms and Plasminogen Activation System in Alzheimer's Disease: Consequences on Extracellular Maturation of Brain-derived Neurotrophic Factor and Therapeutic Implications" *CNS NEUROSCIENCE & THERAPEUTICS*, Vol. 25, No. 3, 31 March 2019 (2019-03-31), pp. 303-313, see the abstract, and figure 1 | 1-7 |

Form PCT/ISA/210 (second sheet) (January 2015)

**INTERNATIONAL SEARCH REPORT**

International application No.

**PCT/CN2021/082720**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a.  ☑ forming part of the international application as filed:

        ☑  in the form of an Annex C/ST.25 text file.

        ☐  on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT

| International application No. |
|---|
| **PCT/CN2021/082720** |

**Box No. II        Observations where certain claims were found unsearchable (Continuation of item 2 of first sheet)**

This international search report has not been established in respect of certain claims under Article 17(2)(a) for the following reasons:

1. ☑ Claims Nos.: **1-15**
because they relate to subject matter not required to be searched by this Authority, namely:

[1]    The subject matter of claims 1-15 relates to methods of treating human or animal body diseases,
and therefore does not warrant an international search according to the criteria set out in PCT Rule
39.1(iv). The subject matter of claim 1 may be reasonably anticipated to be modified to: the use of one
or more compounds in the preparation of a drug that promotes the degradation of misfolded proteins
and aggregates thereof, wherein said compounds are selected from components of the fibrinolysinogen
activation pathway, compounds capable of directly activating fibrinolysinogen or indirectly activating
fibrinolysinogen by activating upstream components of the fibrinolysinogen activation pathway,
compounds that mimic compounds that mimic the activity of fibrinolysinogen or fibrinolytic enzymes,
compounds that upregulate the expression of fibrinolysinogen or fibrinolysinogen activators,
fibrinolysinogen analogs, fibrinolytic enzyme analogs, tPA or uPA analogs, and antagonists of
fibrinolytic inhibitors. The international search is made on the basis of this reasonable anticipation.

2. ☐ Claims Nos.:
because they relate to parts of the international application that do not comply with the prescribed requirements to such an
extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/082720**

| Patent document cited in search report | | | Publication date (day/month/year) | Patent family member(s) | | | Publication date (day/month/year) |
|---|---|---|---|---|---|---|---|
| WO | 0158476 | A2 | 16 August 2001 | AU | 3173301 | A | 20 August 2001 |
| | | | | WO | 0158476 | A3 | 28 March 2002 |
| US | 2014186423 | A1 | 03 July 2014 | | None | | |
| WO | 03071267 | A1 | 28 August 2003 | US | 7057086 | B2 | 06 June 2006 |
| | | | | US | 2003217371 | A1 | 20 November 2003 |
| | | | | EP | 1485709 | A4 | 21 September 2005 |
| | | | | CA | 2476761 | A1 | 28 August 2003 |
| | | | | AU | 2003215315 | A1 | 09 September 2003 |
| | | | | EP | 1485709 | A1 | 15 December 2004 |
| US | 6471960 | B1 | 29 October 2002 | US | 6136548 | A | 24 October 2000 |
| WO | 0162799 | A2 | 30 August 2001 | EP | 1257582 | B1 | 29 April 2009 |
| | | | | WO | 0162799 | A3 | 04 April 2002 |
| | | | | AT | 429926 | T | 15 May 2009 |
| | | | | EP | 1257582 | A2 | 20 November 2002 |
| | | | | US | 2006270599 | A1 | 30 November 2006 |
| | | | | DE | 60138524 | D1 | 10 June 2009 |
| | | | | US | 2003050245 | A1 | 13 March 2003 |
| | | | | AU | 4126201 | A | 03 September 2001 |
| | | | | CA | 2400823 | A1 | 30 August 2001 |
| | | | | EP | 1130031 | A1 | 05 September 2001 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102154253 A
- WO 9704801 A
- US 3773919 A
- EP 58481 A

### Non-patent literature cited in the description

- **RUBINSZTEIN DC.** The roles of intracellular protein-degradation pathways in neurodegen-eration. *Nature,* 2006, vol. 443 (7113), 780-786
- **BRAAK H, BRAAK E.** *Evolution of neuronal changes in the course of Alzheimer's disease, Neural Transm Supp,* 1998, vol. 53, 127-140
- **SPILLANTINI ; SCHMIDT ML ; LEE VM et al.** Alpha-synuclein in Lewy bodies. *Nature,* 1997, vol. 3 88 (6645), 839-840
- **MIZUNO Y ; FUJITA Y ; TAKATAMA M et al.** Peripherin partially localizes in Bunina bodies in amyotrophic lateral sclerosis. *J Neurol Sci,* 2011, vol. 302(1/2), 14-18
- **NY, A., LEONARDSSON ; G., HAGGLUND ; A.C, HAGGLOF ; P., PLOPLIS ; V.A., CARMELIET ; P. AND NY, T.** Ovulation inplasminogen-deficient mice. *Endocrinology,* 1999, vol. 140, 5030-5035
- **SILVERSTEIN RL ; LEUNG LL ; HARPEL PC ; NACHMAN RL.** Complex formation of platelet thrombospondin with plasminogen. Modulation of activation by tissue activator. *J. Clin. Invest.,* November 1984, vol. 74 (5), 1625-33
- **GRAVANIS I ; TSIRKA SE.** Tissue-type plasminogen activator as a therapeutic target in stroke. *Expert Opinion on Therapeutic Targets,* February 2008, vol. 12 (2), 159-70
- **GEIGER M ; HUBER K, WOJTA ; J ; STINGL L ; ESPANA F ; GRIFFIN JH, BINDER BR.** Complex formation between urokinase and plasma protein C inhibitor in vitro and in vivo. *Blood,* August 1989, vol. 74 (2), 722-8
- **R B ; MUKHAMETOVA L 1.** Structure and function of plasminogen/plasmin system [J. *Journal of Bioorganic Chemistry,* 2014, vol. 40 (6), 590-605
- **BARANY et al.** Solid-Phase Peptide Synthesis; The Peptides: Analysis, Synthesis, Biology. *Special Methods in Peptide Synthesis,* vol. 2, 3-284
- **MERRIFIELD.** Solid Phase Peptide Synthesis. I. The Synthesis of a Tetrapeptide. *J. Am. Chem. Soc.,* 1963, vol. 85, 2149-2156
- **STEWART et al.** Solid Phase Peptide Synthesis. Pierce Chem. Co, 1984
- **GANESAN A.** *Mini Rev. Med Chem,* 2006, vol. 6, 3-10
- **CAMARERO JA et al.** *Protein Pept Lett.,* 2005, vol. 12, 723-8
- Suitable mammal host cells include a CHO cell line, various Cos cell line, HeLa cells, a myeloma cell line, and transformed B cells or hybridoma. **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- Remington's Pharmaceutical Sciences. 1980
- **LANGER et al.** *J. Biomed. Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547
- **KENNETHC ROBBINS ; LOUIS SUMMARIA ; DAVID ELWYN et al.** Further Studies on the Purification and Characterization of Human Plasminogen and Plasmin. *Journal of Biological Chemistry,* 1965, vol. 240 (1), 541-550
- **SUMMARIA L ; SPITZ F ; ARZADON L et al.** Isolation and characterization of the affinity chromatography forms of human Glu- and Lysplasminogens and plasmins. *J Biol Chem.,* 25 June 1976, vol. 251 (12), 3693-9
- **HAGAN JJ ; ABLONDI FB ; DE RENZO EC.** Purification and biochemical properties of human plasminogen. *J Biol Chem.,* April 1960, vol. 235, 1005-10
- **WON-SEOK CHOI et al.** Conditional deletion of Ndufs4 in dopaminergic neurons promotes Parkinson's disease-like nonmotor symptoms without loss of dopamine neurons. *Scientific Reports*
- **KALIA L V ; KALIA S K.** Alpha- Synuclein and Lewy pathology in Parkinson's disease. *Curr Opin Neurol,* 2015, vol. 28 (4), 375-381
- **SELKOE D J.** Alzheimer's disease: genes, proteins, and therapy. *Physiol. Rev,* 2001, vol. 81 (2), 741-766
- **NASERI NN ; WANG H ; GUO J ; SHARMA M ; LUO W.** The complexity of tau in Alzheimer's disease. *Neurosci Lett.,* 13 July 2019, vol. 705, 183-194

- **GRAY K ; ELLIS V.** Activation of pro-BDNF by the pericellular serine protease plasmin. *Febs Letters,* 2008, vol. 582 (6), 907-910
- **KOWIAŃSKI, PRZEMYS?AW ; LIETZAU G ; CZUBA E et al.** BDNF: A Key Factor with Multipotent Impact on Brain Signaling and Synaptic Plasticity. *Cellular & Molecular Neurobiology,* 2017
- **ALOE L ; ROCCO M L ; BIANCHI P et al.** Nerve growth factor: from the early discoveries to the potential clinical use. *Journal of Translational Medicine,* 2012, vol. 10 (1
- **LEWIN G R ; LECHNER S G ; SMITH E S J.** *Nerve Growth Factor and Nociception: From Experimental Embryology to New Analgesic Therapy,* 2014
- **ANZAI I ; TOKUDA E ; HANDA S ; MISAWA H ; AKIYAMA S ; FURUKAWA Y.** Oxidative misfolding of Cu/Zn-superoxide dismutase triggered by non-canonical intramolecular disulfide formation. *Free Radic Biol Med,* 01 February 2020, vol. 147, 187-199
- **ZHAO J ; BOYD AW ; BARTLETT PF.** The identification of a novel isoform of EphA4 and ITS expression in SOD1G93A mice. *Neuroscience,* 07 April 2017, vol. 347, 11-21